# EUROPEAN PATENT SPECIFICATION

(11) **EP 3 520 870 B1**
(45) Date of publication and mention of the grant of the patent: **28.04.2021**
(21) Application number: 19154609.2
(22) Date of filing: 30.01.2019
(51) Int. Cl.: B01D 19/00, A61M 1/14

(54) **DEGASSING SYSTEM FOR DIALYSIS**
ENTGASUNGSSYSTEM FÜR DIALYSE
SYSTÈME DE DÉGAZAGE POUR DIALYSE

(30) Priority: 31.01.2018 US 201815885738
(43) Date of publication of application: 07.08.2019
(73) Proprietor: Medtronic, Inc., Minneapolis, MN 55432 (US)
(72) Inventor: MEYER, Thomas E., Stillwater, Minnesota 55082 (US); SCHMIDT, Samuel J., Brooklyn Center, Minnesota 55430 (US); HAIJKO, William P., Safety Harbor, Florida 34695 (US); GOMES, Carl Wilbert, Parrish, Florida 34219 (US)
(74) Representative: Maschio, Antonio

(56) References cited:
- GB-A- 2 479 130
- US-A- 3 932 150
- US-A- 5 180 403
- US-A- 5 203 890
- US-A1- 2003 034 305
- US-A1- 2007 140 916
- US-A1- 2009 084 718
- US-A1- 2011 168 017

## Description

### CROSS-REFERENCE TO RELATED APPLICATIONS

This application is a Continuation-in-part of U.S. Patent Application No. 14/566,686 filed December 10, 2014, now U.S. Patent No. 9,713,665.

This application also claims benefit of and priority to U.S. Patent Application No. 15/618,187 filed June 9, 2017, which is a Continuation of U.S. Patent Application No. 14/566,686 filed December 10, 2014, now U.S. Patent No. 9,713,665.

### FIELD OF THE INVENTION

The invention relates to a degassing vessel and related systems and methods that can remove certain gases such as carbon dioxide from a dialysis system with minimal foaming inside the degassing vessel. The invention further relates to mechanical systems and methods for degassing a dialysate or any fluid used for, during or resulting from dialysis.

### BACKGROUND

In dialysis systems including sorbent based systems, gas such as carbon dioxide can be created as part of sorbent dialysis. In particular, carbon dioxide can be generated as the gas is formed during urea breakdown as spent dialysate flows through a sorbent cartridge. The bicarbonate buffer system can also contribute to the creation of excess carbon dioxide in dialysate. Further, dialysate can contain dissolved oxygen and nitrogen gas that crosses the dialysis membrane from the patient's blood. Dissolved gases such as nitrogen and oxygen can also be present in water that is used to initially prepare a dialysate. The resulting gases from any one of the sources can go into solution in dialysate and form gas bubbles.

Removal of carbon dioxide and other dissolved and undissolved gases can be important for maintaining a required pH or maintaining certain fluid conditions such as a bicarbonate or ion concentration. For example, a desired partial pressure of carbon dioxide may be required for safe operation during dialysis. Further, excesses gases can be removed to avoid creating gas bubbles. Gas bubbles can interfere with the smooth pumping of dialysate in a dialysate loop and can interfere with sensors in the dialysate flow path and reduce diffusive clearance across the dialysis membrane. Gas bubbles can also result in a dangerous condition if gas crosses the dialyzer membrane into an extracorporeal circuit and creates gas bubbles in blood returning to a patient. Known systems suffer from excess foaming within the degassing vessel, which can reduce accuracy in measuring fluid levels and impede fluid flow through the degassing system.

The degassers known in the art (e.g. US2003/0034305A1) oftentimes fail to efficiently remove dissolved gases, such as carbon dioxide, from fluid, or do not provide control over the amount of carbon dioxide removed. Hence, there is a need for a degasser that can remove large amounts of dissolved carbon dioxide from solution, while providing control over the amount of dissolved and undissolved gases removed from fluid before, during and after dialysis therapy. There is also a need for a degasser having the small size and weight necessary for a portable device. There is a further need for a degassing system that can reduce foaming within the degassing vessel.

### SUMMARY OF THE INVENTION

The first aspect of the invention is drawn to a degassing vessel. In the invention according to claim 1, the degassing vessel comprises a fluid inlet in the degassing vessel fluidly connected to a dialysate flow path; a liquid outlet in the degassing vessel fluidly connected to the dialysate flow path; a gas outlet fluidly connected to a vacuum pump; and a degas sprayer fluidly connected to the fluid inlet.

The degas sprayer can direct liquid downwardly into the degassing vessel.

The degassing vessel comprises a spray chamber and a float chamber; the spray chamber fluidly connected to the float chamber; wherein the degas sprayer is positioned above the spray chamber.

In any embodiment, the spray chamber can have a substantially conical shape.

The liquid outlet is positioned in a bottom portion of the spray chamber.

In any embodiment, the gas outlet can be positioned between the spray chamber and the float chamber.

The degassing vessel comprises a level sensor in the float chamber.

In any embodiment, the level sensor can comprise one or more of: a float with a magnet and a linear array of Hall effect sensors, an ultrasonic sensor, and a capacitive sensor.

In any embodiment, the degassing vessel can comprise a temperature sensor in the liquid outlet.

In any embodiment, the degassing vessel can comprise a pressure sensor in the gas outlet.

In any embodiment, the degassing vessel can comprise a valve positioned between the gas outlet and the vacuum pump.

In any embodiment, the degassing vessel can comprise a vent valve positioned between a vent and the gas outlet.

Any of the features disclosed as being part of the first aspect of the invention can be included in the first aspect of the invention, either alone or in combination.

The system comprises a dialysate flow path comprising a degassing flow loop; the degassing flow loop comprising a degassing vessel having a fluid inlet fluidly connected to a degas sprayer at a top portion of the degassing vessel, a liquid outlet at a bottom portion of the degassing vessel, and a gas outlet at a top portion of the degassing vessel; a vacuum pump fluidly connected to the gas outlet; a first fluid line fluidly connecting the liquid outlet to a second fluid line fluidly connected to the fluid inlet, the first fluid line comprising a fluid pump; a third fluid line fluidly connecting the second fluid line to the dialysate flow path; and a controller controlling the vacuum pump and the fluid pump.

In any embodiment, the degassing flow loop can be parallel to the dialysate flow path; and a flow rate of the degassing flow loop can be controlled independently of a flow rate of the dialysate flow path.

In any embodiment, the system can comprise a first pressure sensor in the degassing flow loop upstream of the fluid inlet and a second pressure sensor in the degassing flow loop in the gas outlet.

In any embodiment, the controller can control a first valve positioned between the vacuum pump and the gas outlet and/or a vent valve positioned between a vent and the gas outlet based on an absolute pressure in a headspace of the degassing vessel.

In any embodiment, the controller can control the first valve and/or the vent valve to maintain a carbon dioxide level in the third fluid line of between 40mmHg and 150mmHg pCO₂ (1mm Hg = 133.322 Pa).

In any embodiment, the system can comprise at least one valve positioned in the third fluid line between the gas outlet and a vent.

In any embodiment, the system can comprise an ambient pressure sensor.

In any embodiment, the degassing vessel can comprise a level sensor in communication with the controller; the level sensor comprising one or more of: a float with a magnet and a linear array of Hall effect sensors, an ultrasonic sensor, and a capacitive sensor.

Any of the features disclosed as being part of the second aspect of the invention can be included in the second aspect of the invention, either alone or in combination.

### BRIEF DESCRIPTION OF THE DRAWINGS

FIG. 1A shows a schematic of a degassing module for use in sorbent dialysis configured to degas dialysate.
FIG. 1B shows a schematic of a degassing module for use in sorbent dialysis configured to allow air to be drawn into the system.
FIG. 2 shows a schematic of a degassing module for use in sorbent dialysis configured to degas dialysate utilizing a nucleation chamber.
FIG. 3 is a graph showing the outlet CO₂ concentration in a degasser as a function of the absolute pressure in the degassing vessel.
FIG. 4 is a graph showing the outlet CO₂ concentration in a degasser as a function of the flow rate in a system with a degasser at ambient pressure.
FIG. 5A is a graph showing the amount of dissolved CO₂ removed by a degasser with a fluid pump upstream of the degassing vessel for two locations in a dialysis circuit.
FIG. 5B is a graph showing the change in pH of a fluid passing through a degasser with a fluid pump upstream of the degassing vessel for two locations in a dialysis circuit.
FIG. 6A is a graph showing the amount of dissolved CO₂ removed by a degasser with a fluid pump downstream of the degassing vessel as a function of the dialysate flow loop flow rate.
FIG. 6B is a graph showing the change in pH of a fluid passing through a degasser with a fluid pump downstream of the degassing vessel as a function of the dialysate flow loop flow rate.
FIG. 7 is a graph showing the amount of dissolved CO₂ removed by a degasser with a fluid pump downstream of the degassing vessel as a function of the degassing flow loop flow rate.
FIG. 8A is a graph showing the amount of dissolved CO₂ removed by a degasser with a fluid pump downstream of the degassing vessel as a function of the vacuum level in the degassing flow loop.
FIG. 8B is a graph showing the change in pH of a fluid passing through a degasser with a fluid pump downstream of the degassing vessel as a function of the vacuum level in the degassing flow loop.
FIG. 9A is a graph showing the amount of dissolved CO₂ removed by a degasser with a fluid pump downstream of the degassing vessel as a function of the CO₂ concentration at the inlet of the degasser.
FIG. 9B is a graph showing the change in pH of a fluid passing through a degasser with a fluid pump downstream of the degassing vessel as a function of the pH at the inlet of the degasser.
FIG. 10 is a flow diagram showing the operation of the pumps in relation to the carbon dioxide present in the dialysate.
FIG. 11 is a flow diagram showing an alternative operation of the pumps in relation to the carbon dioxide present in the dialysate.
FIG. 12 is a schematic of a degassing system having a pressure sensor to measure the pressure within the degasser; and having control valves to alternately connect the vent port of the degassing vessel to an air inlet filter, a drain line for gas removal through a vacuum pump, or a dialysate flow path for recirculation of fluid.
FIG. 13 shows a degassing vessel according to the claimed invention with a degas sprayer entering through a top of
   the degassing vessel.
FIG. 14 shows a cross-sectional view of a degassing vessel.
FIG's 15A-B show top and side views of a degassing vessel.
FIG. 16 shows a non-limiting embodiment of a spray nozzle for use in a degassing system.
FIG.'s 17A-B show expected carbon dioxide levels entering a degassing system based on simulated treatments.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

The term "absolute pressure" refers to a pressure of a liquid, gas, or combination thereof, relative to a vacuum.

An "ambient pressure sensor" is a pressure sensor positioned to measure a pressure outside of a container, system, or fluid line, such as atmospheric pressure.

The term "bottom portion" refers to a portion of a component at a height lower than the center of the component when positioned for normal use.

A "capacitive sensor" is a sensor that measures distance to a conductive object by measuring changes in capacitance as the conductive object closer to or further away from the sensor.

The term "carbon dioxide sensor" refers to devices that can detect or measure the concentration of carbon dioxide in a fluid, gas, or combination thereof.

The terms "communicate" and "communication" include, but are not limited to, the connection of system electrical elements, either directly or remotely, for data transmission among and between said elements. The terms also include, but are not limited to, the connection of system fluid elements enabling fluid interface among and between said elements.

The term "comprising" includes, but is not limited to, whatever follows the word "comprising." Thus, use of the term indicates that the listed elements are required or mandatory but that other elements are optional and may or may not be present.

A "conical shape" or "substantially conical shape" refers to a three-dimensional shape of a component that has a larger diameter on a first side than on a second side and inwardly or outwardly tapering walls connecting the first side and second side.

The term "consisting of" includes and is limited to whatever follows the phrase "consisting of." Thus, the phrase indicates that the limited elements are required or mandatory and that no other elements may be present. The term "consisting essentially of" includes whatever follows the term "consisting essentially of" and additional elements, structures, acts or features that do not affect the basic operation of the apparatus, structure or method described.

The terms "control," "controlling," or "controls" can refer to the ability of one component to direct the actions of a second component.

The term "controlled independently" refers to the ability to vary one parameter of a system without varying a second parameter of the system.

A "controller," "controller," "processor," or "microprocessor" is a device which monitors and affects the operational conditions of a given system. The operational conditions are typically referred to as output variables of the system wherein the output variables can be affected by adjusting certain input variables.

A "degas sprayer" is a component that atomizes or increases the surface area to volume ratio of a fluid.

A "degasser" is a component that is capable of removing dissolved and undissolved gasses from fluids. The term "degasser" can encompass a degassing vessel, and a fluid pump and a vacuum pump connected to the degassing vessel and working in concert to create a vacuum in the fluid flowing through the degassing vessel and to evacuate gas from the degassing vessel.

A "degassing flow loop" is a portion of a fluid pathway that conveys a dialysate from a dialysate flow loop to a degasser and back to the dialysate flow loop.

A "degassing vessel" or a "degas vessel" is a component of a degasser, and can be any structure having an inlet through which fluid enters the vessel, a first outlet through which gas removed from the fluid may pass, and a second outlet through which fluid can exit the vessel.

The term "dialysate flow loop," "dialysate flow path" or "dialysate conduit flow path" refers to any portion of a fluid pathway that conveys a dialysate and is configured to form at least part of a fluid circuit for hemodialysis, hemofiltration, ultrafiltration, hemodiafiltration or ultrafiltration. Optionally, the fluid pathway can contain priming fluid during a priming step or cleaning fluid during a cleaning step.

"Dialysis" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

The term "downstream" refers to a position of a first component in a flow path relative to a second component wherein fluid, gas, or combinations thereof, will pass by the second component prior to the first component during normal operation. The first component can be said to be "downstream" of the second component, while the second component is "upstream" of the first component.

The terms "downward" or "downwardly" refer to a direction from a higher elevation to a lower elevation when the system is configured for normal use.

A "float" is a component with a density lower than that of a fluid, causing the float to raise to the top of the fluid.

A "float chamber" is a chamber or other portion of a component that contains a fluid level sensor. In certain embodiments, the fluid level sensor can operate using a float located within the float chamber.

"Flow rate" refers to a volume of a fluid, gas, or combination thereof, moved per unit time.

The term "fluidly connectable" refers to the ability to provide passage of fluid, gas, or combinations thereof, from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type.

The term "fluidly connected" refers to a particular state or configuration of one or more components such that fluid, gas, or combination thereof, can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "fluid inlet" refers to a conduit or opening through which fluid, gas, or a combination thereof, can enter a component or apparatus.

A "fluid line" can refer to a tubing or conduit through which a fluid, gas, or a combination thereof can pass. The fluid line can also contain air during different modes of operation such as cleaning or purging of a line.

A "fluid pump" is a pump used to move fluid, gas, or a combination thereof, throughout a system.

The term "gas outlet" refers to a conduit or opening through which gas can exit a component or apparatus. In certain embodiments, a gas outlet can also allow fluids to enter or exit the component.

The term "headspace" refers to a portion of a container or vessel containing air that is above a liquid.

A "level sensor" is a component capable of determining the level of a fluid in a container. The terms "upper level sensor" and "lower level sensor" refer to the respective positions of level sensors.

A "linear array of Hall effect sensors" is a set of components that measure a magnetic field in order to measure a distance to a magnet. In certain embodiments, the linear array can include multiple Hall sensors in a vertical line, each sensor measuring a distance to the magnetic object in order to calculate the position of the magnetic object.

The term "liquid outlet" refers to a conduit or opening through which liquid can exit a component or apparatus. In certain embodiments, a gas can exit the component through the liquid outlet during cleaning, disinfection, or set up of the component.

A "magnet" is a material capable of creating a magnetic field around itself.

The term "maintain a carbon dioxide level" refers to controlling a system to prevent the concentration of carbon dioxide in a fluid from substantially deviating from a predetermined value or range.

The term "parallel," as used to describe two or more flow paths, refers to a configuration wherein fluid, gas, or a combination thereof can only travel through one of the two or more flow paths without being recirculated.

The terms "pressure meter" and "pressure sensor" refer to a device for measuring the pressure of a gas, a fluid, or a combination thereof in a vessel, container, or fluid line.

A "spray chamber" is a chamber or other portion of a component into which a fluid can be sprayed.

The term "temperature sensor" refers to a device for measuring the temperature of a fluid, a gas, or a combination thereof in a vessel, container, or fluid line.

The term "top portion" refers to the portion of a component at a height higher than the center of a component when positioned for normal use.

An "ultrasonic sensor" is a sensor that measures distance to an object by determining a length of time necessary for an ultrasonic wave to reach the object and reflect back to the sensor.

The term "upstream" refers to a position of a first component in a flow path relative to a second component, wherein fluid, gas, or a combination thereof, will pass by the first component prior to the second component during normal operation. The first component can be said to be "upstream" of the second component, while the second component is "downstream" of the first component.

A "vacuum pump" is a pump used to create negative pressure in a component.

A "valve" is a device capable of directing the flow of fluid, gas, or a combination thereof, by opening, closing or obstructing one or more pathways to allow the fluid, gas, or combination thereof to travel in a particular path. One or more valves configured to accomplish a desired flow can be configured into a "valve assembly."

A "vent valve" is a valve that controls the movement of a gas into and out of a vent. In certain embodiments, a vent valve can also allow fluids to enter or exit the vent.

The term "vent" as referred to in relationship to a gas, refers to a means for permitting the escape of a gas from a defined portion of a system, vessel, container, or fluid line. In certain embodiments, fluids may also escape through the vent.

### Degassing Vessel

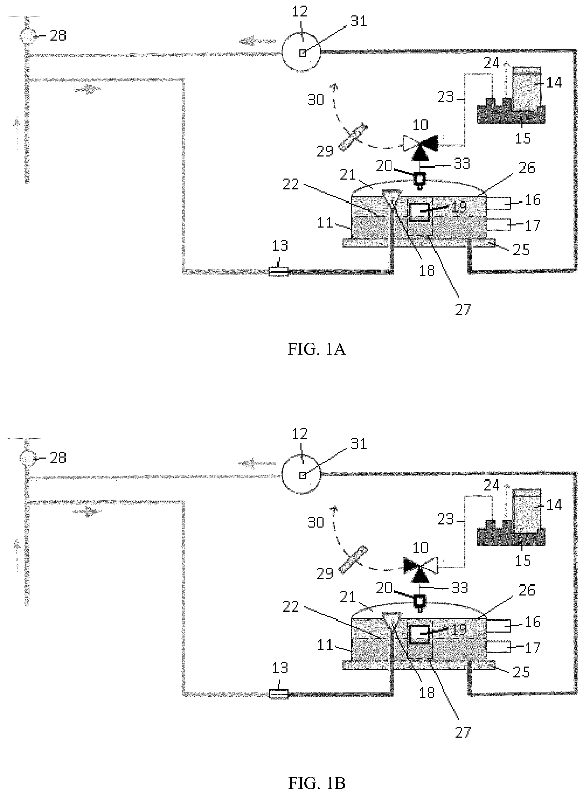

The aspects of the disclosure relate to a degasser and related systems and methods for removing gas, and specifically carbon dioxide, generated from the breakdown of urea in the sorbent cartridge. A degassing module is shown in FIG. 1A. The direction of dialysate flow is shown by the arrows. The degassing module can be placed in the dialysis circuit preferably at a point between the sorbent cartridge (not shown) and the dialyzer (not shown). The degassing module can have a degassing flow loop providing fluid flow that is in parallel to the dialysate flow path. The parallel configuration allows the fluid flow through the degassing loop to be independent of the fluid flow rate through the dialyzer such that the fluid flow rate through the degassing loop can be either less than or greater than the dialysate flow rate through the dialyzer. Thus, the parallel configuration provides control flexibility to adjust the degassing loop flow rate for optimal degassing without requiring the dialysate flow rate through the dialyzer to change. Alternatively, the fluid flow through the degassing module can be arranged in series with the dialysate flow to the dialyzer.

As the dialysate enters the degassing module, the dialysate can pass a degas restrictor **13** of FIG. 1A. The degas restrictor **13** can serve to restrict the flow of fluid through the degassing system. The degas restrictor **13** may be a narrow tube or any portion of the flow path that can be narrowed in a controlled fashion. For example, restriction can be provided by a portion of the flow path being crushable and having roller portions to create a portion of the flow path having a narrowed inner diameter to thereby restrict flow. Any other mechanical structures known to those of ordinary skill to restrict flow is also contemplated by the disclosure. The fluid pump **12,** fluidly connected to the degas restrictor **13,** can pull fluid through the degas restrictor **13,** creating a reduced pressure in the degassing vessel **11** side of the degas restrictor **13.** The fluid pump **12** provides energy necessary to remove the degassed liquid from the low pressure of the degassing vessel **11** and return the degassed liquid to the higher pressure of the main dialysate flow path. A vacuum can be created in the degassing vessel **11** side of the degas restrictor **13.** A pressure sensor (not shown) can be placed after the degas restrictor **13** to determine the pressure of fluid in the degasser. Importantly, the fluid pump **12** of the present disclosure can be located downstream of the degassing vessel **11** to allow for improved removal of carbon dioxide. The vacuum that can be created by pulling the fluid through the degas restrictor **13** helps to draw dissolved gases, including carbon dioxide, out of solution by reducing the pressure of the fluid below the partial pressure of the dissolved gas in the liquid. The degas restrictor **13** need not be a separate component. Instead, the fluid inlet of the degassing vessel **11** can be narrow, and therefore operate as a flow restrictor. Vacuum pump **14** on the gas removal pump assembly **15** can be fluidly connected to the degassing vessel **11** by gas removal line **23** and can desirably remove the gases in the low pressure environment inside degassing vessel **11** via mechanical vent valve **20.** The fluid enters the degassing vessel **11,** by crossing through the base **25** of the degassing vessel **11** and through degas sprayer **18.** However, there is no particular requirement of the first, second or third aspects of the invention for the fluid to enter or exit through the base. The degas sprayer **18** creates a thin spray or mist, which can increase release of dissolved gases from solution by increasing the surface area of liquid in contact with the low pressure atmosphere in the gas space **21** inside degassing vessel **11** to increase the rate at which gas can be liberated from the liquid. In certain embodiments, the fluid can enter the degassing vessel **11** at other locations than the base **25.** For example, fluid can enter the degassing vessel **11** at a location on the side of the degassing vessel **11.** The degas sprayer **18** can be positioned within the degassing vessel **11** so that the degas sprayer **18** is above the maximum fluid level **26.** The degas sprayer **18** is optional and not required to remove carbon dioxide or other gases from the dialysate solution. Flow restrictions in degas sprayer **18** cause sufficient pressure reduction in the fluid and degas restrictor **13** is not required. Carbon dioxide and other gases collect in the gas space **21** of the degassing vessel **11** and leave the degassing vessel **11** through vent valve **10,** positioned on a connector **33** fluidly connected to the degassing vessel **11.** Although depicted as a 3-way valve, vent valve **10** can be any combination of one or more valves suitable for accomplishing the desired control of gas flow. In FIG. 1A, the pathways open in vent valve **10** are shown in black. Vacuum pump **14** on the gas removal pump assembly **15** is attached to the degassing vessel **11** by gas removal line **23,** and provides the force necessary to move gases from the lower pressure degassing vessel **11** out into the atmosphere. The vacuum pump **14** exerts a vacuum that is greater than or equal to the vacuum created by the fluid pump **12** pulling fluid through the degas restrictor **13,** which allows the removal of the accumulated gas from the degassing vessel **11.**

The degassing vessel **11** can be operated at a pressure lower than atmospheric pressure due to the presence of vacuum pump **14.** By maintaining the degassing vessel **11** at a pressure less than atmospheric pressure, carbon dioxide present in the fluid can be more easily removed than in the absence of the described system of pumps. The vent valve **10** can allow gas to leave directly into the atmosphere through filter **29,** as represented by arrow **30.** The filter **29** is a particle filter that serves to remove particulate matter from air flowing through filter **29.** The gases may travel through gas removal line **23,** to the gas removal pump assembly **15** and into the atmosphere as represented by arrow **24.**

Vent valve **10** can be a three way valve, as shown in FIG. 1A. This can allow air to be removed from the degassing vessel **11** through the gas removal line **23,** and also allow air to be drawn into the degas flow loop when fluid is being drained from the system. Overflow float **19** and mechanical vent valve **20** can provide a mechanism for an automatic shutdown, preventing fluid from leaving the degassing vessel **11** through the vent valve **10,** but allowing air to be added or removed during filling or draining of the system. If the fluid level in the degassing vessel **11** reaches above a certain point, overflow float **19** can block, either directly or indirectly, the fluid from passing through mechanical vent valve **20.** The maximum fluid level in the degassing vessel **11** can be shown by line **26,** while the minimum fluid level can be shown by line **22.** A degas float channel **27** can be used to ensure that the overflow float **19** properly engages with the mechanical vent valve **20.** The degas float channel **27** can be placed directly underneath the mechanical vent valve **20** so that when the overflow float **19** rises to the top of the degassing vessel **11,** the overflow float **19** will properly cover the mechanical vent valve **20.** Alternatively, the float can move an actuator so that the mechanical vent valve **20** is closed. The degas float channel **27** can be made with a fluid permeable substance, such as mesh, so that fluid can still move freely through the degassing vessel **11.** In certain embodiments, the function of the degas float channel **27** can be accomplished by a rod through the overflow float **19** wherein the rod is anchored to the degassing vessel **11.** The overflow float **19** can be tethered to actuators (not shown). If the overflow float **19** rises, the tethers (not shown) can activate the actuators by pulling on the actuators to either shut off, or modulate the pump rate of, the vacuum pump **14** and fluid pump **12.**

Lower level sensor **17** and upper level sensor **16** can sense the fluid level in the degassing vessel **11.** The fluid level in the degassing vessel **11** can be a function of the vacuum created by fluid pump **12** and vacuum pump **14** working independently or in concert. The pump rate of the fluid pump **12** and vacuum pump **14** can be adjusted as necessary to maintain the correct fluid level in the degassing vessel **11.** The lower level sensor **17** and upper level sensor **16** can be in electronic communication with a controller (not shown). The pump rates of the fluid pump **12** and vacuum pump **14** can be automatically adjusted by the controller to maintain the proper level of fluid in the degassing vessel **11.** If the fluid level in the degassing vessel **11** is near or above the maximum fluid level **26,** the pump rates of the fluid pump **12** can be increased, and/or vacuum pump **14** can be reduced. If the fluid level in the degassing vessel **11** is near or below the minimum fluid level **22,** the pump rates of the fluid pump **12** can be reduced and/or vacuum pump **14** can be increased.

In certain embodiments, only one sensor is necessary to detect the fluid level in the degassing vessel **11.** For example, an ultrasonic sensor or mechanical float can be used to determine the fluid level in the degassing vessel **11.** Any other type of fluid level sensor known in the art is contemplated.

Carbon dioxide sensor **28** can determine the amount of carbon dioxide present in the dialysate flow path after dialysate has passed through the degasser. The pump rates of fluid pump **12** and vacuum pump **14** can be adjusted as discussed below in response to signals received from the carbon dioxide sensor **28** in order to remove more or less carbon dioxide from the dialysate, and therefore deliver more or less carbon dioxide to the main dialysate flow path. The pumps can be adjusted automatically if the level of carbon dioxide detected in the dialysate by carbon dioxide sensor **28** is higher or lower than a pre-set value. Alternatively, the pumps can be adjusted manually in response to output from the carbon dioxide sensor **28.** The system can control the degasser to maintain a carbon dioxide level in fluid exiting the degasser between any of 50 and 200 mmHg partial pressure, 50 and 120 mmHg partial pressure, 50 and 80 mmHg partial pressure, 70 and 100 mmHg partial pressure, 80 and 120 mmHg partial pressure, 50 and 200 mmHg partial pressure, or 100 and 200 mmHg partial pressure. The carbon dioxide sensor **28** can be placed anywhere in the dialysate flow path, but preferably between the outlet of the degassing flow path and the inlet of the dialyzer (not shown). One of skill in the art will understand that the carbon dioxide sensor **28** can be any components capable of measuring the carbon dioxide in a fluid, directly or indirectly.

Carbon dioxide sensors and sensors are known in the art. Examples include non-dispersive infrared (NDIR) detectors that detect carbon dioxide concentration in a gas and which are commercially available from a number of manufacturers, for example Gas Sensing Solutions, Glasgow Scotland; colorimetric optical detectors that detect carbon dioxide in a liquid by means of a substrate that produce color change when the concentration of carbon dioxide in the liquid changes (PreSens Precision Sensing GmbH, Regensburg Germany); and sensors that utilize Severinghaus electrodes, such as the InPro CO₂ sensor from Mettler Toledo, Leicester England.

The pumps of the degassing module can be of any type known in the art. In certain embodiments, fluid pump **12** and vacuum pump **14** can be the same type of pump. Alternatively, fluid pump **12** and vacuum pump **14** may be different types of pumps. In certain embodiments, the fluid pump **12** and vacuum pump **14** can be a gear pump. Alternatively, fluid pump **12** and vacuum pump **14** can be a peristaltic pump, a diaphragm pump or an impeller pump. Fluid pump **12** can also have a sensor **31** attached to the fluid pump **12** to monitor performance of the fluid pump **12** and detect wear. The fluid pump **12** must be selected for operating with the pump inlet at a low absolute pressure necessary to efficiently remove carbon dioxide.

Flow of fluid through the degassing module can be variable. Control over the flow can be provided by fluid pump **12.** Under certain operating conditions the flow rate provided by fluid pump **12** can be less than the flow rate through the main dialysate loop. Fluid pump **12** can be operated so that flow through the degassing module is significantly greater than flow through the main dialysate loop. Fluid pump **12** can be operated to move fluid through the degassing flow loop at a rate of 2-3 times that of the dialysate flow path. Alternatively, the fluid pump **12** can be operated to move fluid through the degassing flow loop at a rate between 1-6 times that of the dialysate flow path, 1-2 times that of the dialysate flow path, 3-4 times that of the dialysate flow path, 4-5 times that of the dialysate flow path or 5-6 times that of the dialysate flow path. The flow through the degassing module can be controlled automatically by a controller in communication with the fluid pump **12** depending on the amount of carbon dioxide that is to be removed.

The disclosure can utilize the vacuum pump **14** to remove gas from the degassing vessel **11** to the atmosphere when the degassing vessel **11** is operated under vacuum. Known degassing systems pump fluid into a vessel at ambient pressure where bubbles are allowed to escape. However, providing a second pump or any one of the specific pump configurations described in the first, second and third aspects of the invention to keep a degassing vessel **11** under vacuum can unexpectedly result in higher amount of gases such as carbon dioxide being removed.

The passage from the degassing vessel **11** to vent valve **10** can be covered by a hydrophobic membrane (not shown). A hydrophobic membrane will prevent fluid from escaping the degassing vessel **11** through mechanical vent valve **20.** This, in turn, protects the vacuum pump **14** from being damaged by liquid and prevents undesired loss of liquid from the system while still enabling gas to be removed. The hydrophobic membrane can be positioned in any appropriate location to guard against inadvertent fluid flow to the vacuum pump **14,** and thereby prevent fluid damage. One example of a hydrophobic membrane is Polytetrafluoroethylene, or PTFE. However, the hydrophobic membrane can be made of any material.

During draining of the dialysis air can be drawn into the system in order to drain out the fluid in the fluid pathways of the system. Air can be added to the system through vent valve **10** as shown in FIG. 1B. In FIG. 1B, the pathways of vent valve **10** that are open are shown in black. Air can be passed through filter **29,** which can remove any particulate matter and microorganisms before the air enters the dialysis system, and into the degassing vessel **11** through vent valve **10.** Fluid pump **12** can force this air into the dialysate flow path (not shown).

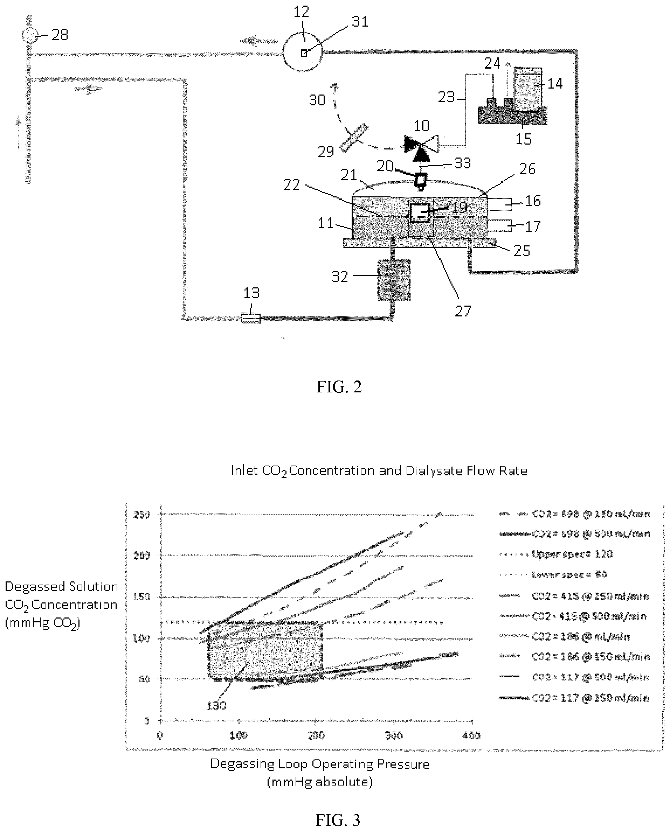

As shown in FIG. 2, the function of the degas sprayer can be replaced by a nucleation chamber **32.** Nucleation chamber **32** contains a high surface area medium, such as fiber mesh, filter or beads, or other configuration known to those of ordinary skill. The high surface area provides sites where gas bubbles can nucleate and collect to form larger bubbles, making removal of the gases more efficient. The bubbles rise through the fluid as the fluid enters the degassing vessel **11** and collect at the gas space **21,** similar to what is shown in FIG. 1A. The nucleation chamber **32** can be placed inside of the degassing vessel **11,** so that fluid moves through the nucleation chamber **32** as the fluid moves through the degassing vessel **11** and gas bubbles, once freed from the high surface area medium in the nucleation chamber **32,** are immediately collected in the gas space **21** of the degassing vessel **11.**

In certain embodiments, both a nucleation chamber and a degas sprayer can be used. Such an arrangement can further help gas to be released from solution to collect at the top of the degassing vessel **11.** However, in certain embodiments, only one of a degas sprayer or nucleation chamber can be used.

FIG. 3 is a graph showing the CO₂ outlet concentration, stated as partial pressures, at the outlet of the degasser as a function of the absolute pressure in the degassing vessel **11** for a variety of CO₂ inlet concentrations, stated as partial pressures. The block labeled **130** is a desired operating CO₂ concentration, expressed as a partial pressure, of between 50 and 120 mmHg. The absolute pressure in the degassing vessel **11** shown in FIG.'s. 1 and 2 is a function of the fluid pressure, determined by the pump rate of the fluid pump **12,** and the vacuum pressure, determined by the pump rate of the vacuum pump **14.** By controlling the two pumps, the pressure in the degassing vessel **11** can be accurately controlled. As shown in FIG. 3, the degasser is capable of removing enough CO₂ to maintain a carbon dioxide level at the outlet of the degasser between 50 and 120 mmHg for a large range of inlet CO₂ concentrations and dialysate flow rates. A degassing vessel pressure of between 60 and 200 mmHg absolute pressure can allow for optimal CO₂ removal across a range of inlet CO₂ concentrations and dialysate flow rates. In certain embodiments, degassing vessel pressure of between any of 40 mmHg and 2000 mmHg, 40 mmHg and 300 mmHg, 40 mmHg and 100 mmHg, 80 mmHg and 150 mmHg, 120 mmHg and 250 mmHg or 200 mmHg and 300 mmHg, can allow for optimal CO₂ removal. The desired outlet concentration of CO₂ can be obtained for the entire range of inlet CO₂ concentrations and flow rates tested by adjusting the pump rates of the two pumps to arrive at the necessary degassing vessel pressure. The vacuum pump **14** may be shut off if the CO₂ concentration is below the lower limit. In such cases, the pressure in the degassing vessel **11** will be the same as the pressure of the dialysate fluid, which can be up to 2000 mmHg.

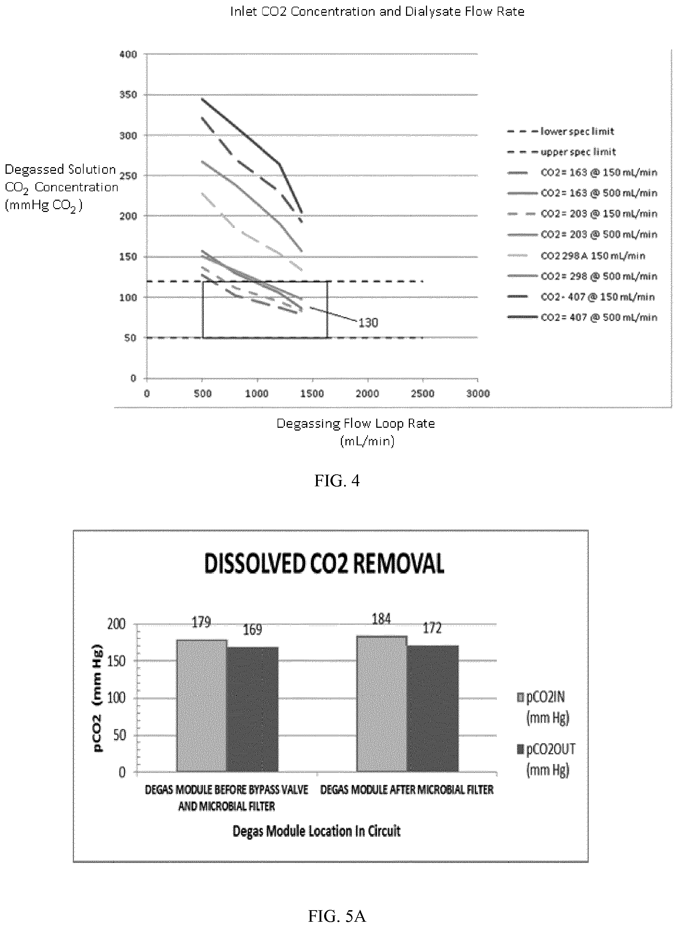

FIG. 4 provides comparative data for known systems operating at ambient pressures showing an outlet CO₂ concentration, stated as partial pressure, in a system that does not use a vacuum pump. Because no vacuum pump is used in known systems, and the known degassing vessels are not able to operate at low absolute pressures, the amount of CO₂ removed is limited by the need to maintain sufficient pressure in the degassing vessel to vent the released gas. As can be seen in FIG. 4, a degasser without a degassing vessel under vacuum can only operate to obtain an outlet CO₂ concentration of between 50 and 120 mmHg when the inlet concentration of CO₂ is around 200 mmHg or below.

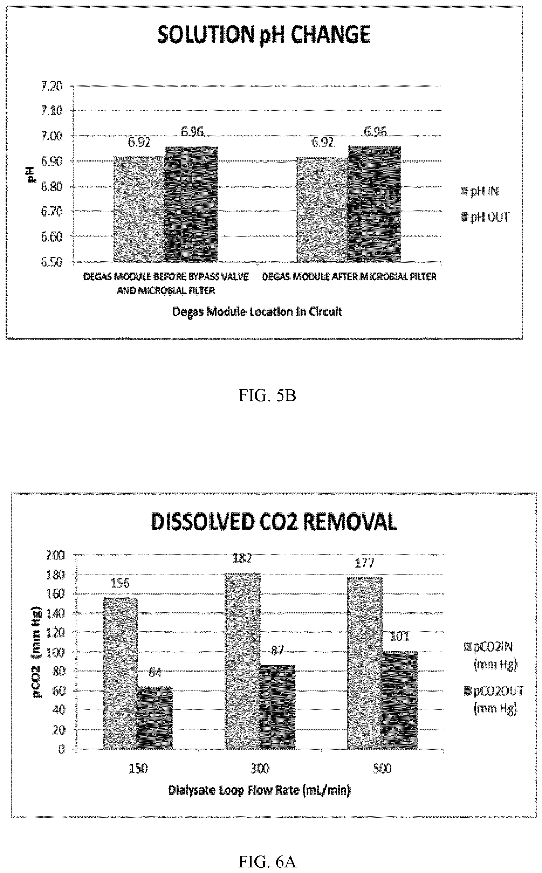

As shown in FIG.'s 5 and 6, the addition of the fluid pump downstream from the degassing vessel can be important to the first, second and third aspects of the invention. By placing the fluid pump downstream of the degas vessel, the efficiency of removing CO₂ was increased. FIG. 5A shows the amount of CO₂ removed from dialysate without operating the degas vessel under vacuum by means of a fluid pump placed downstream of the degas vessel. FIG. 5B shows the change in pH in the same system. By contrast, FIG.'s 6A and 6B show the amount of CO₂ removed, and the effect on pH, in the same system with a fluid pump added downstream of a degassing vessel, shown for a dialysate flow path flow rate from 150 mL/min to 500 mL/min. As can be seen in FIG.'s 6A and 6B, by adding the fluid pump to a downstream location, between 1/3 and 2/3 of CO₂ can be removed, depending on the dialysate flow rate. By contrast, as shown in FIG.'s 5A and 5B, much less CO₂ is removed when the fluid pump is placed upstream of a degas vessel.

As can be seen in FIG. 5, the location of the degasser upstream or downstream with respect to a microbial filter does not alter the amount of CO₂ removed. The described configuration with degasser upstream of the microbial filter can provide for the removal of gas from the dialysate prior to reaching the microbial filter, and thereby advantageously reduce gas accumulation in the microbial filter.

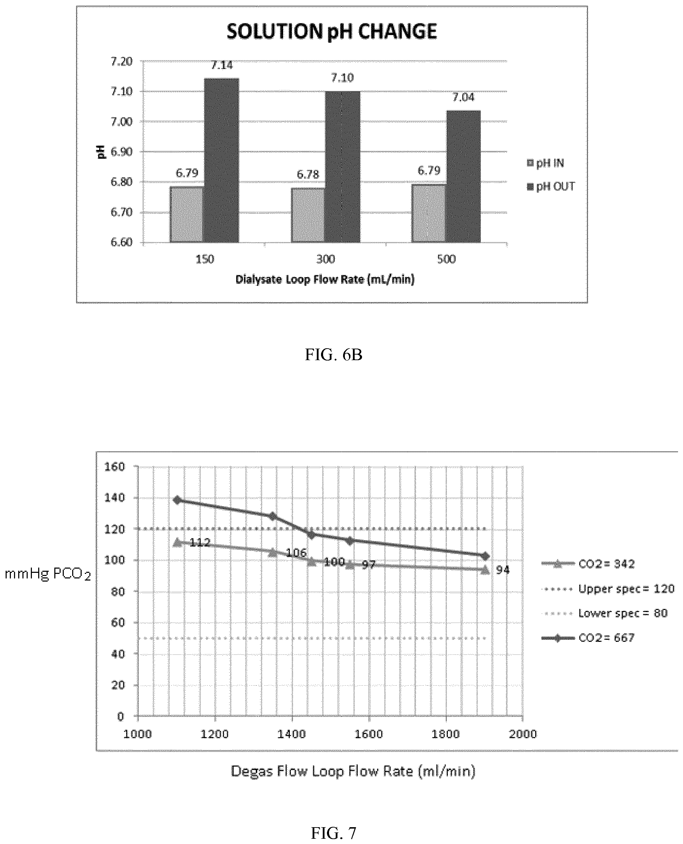

FIG. 7 shows the amount of CO₂ removed as a function of the rate of flow through the degassing flow loop. In all runs shown in FIG. 7 the dialysate flow rate was 600 mL/min. As is shown, the amount of CO2 removed can increase as the flow rate through the degassing flow loop increases.

FIG.'s 8A and 8B show the amount of CO₂ removed, and the effect on pH, as a function of the absolute pressure in the degassing flow loop. In these trials, the dialysate flow rate and degassing flow rate were held constant at 300 mL/min. As can be seen, more CO₂ is removed as the absolute pressure in the degassing flow loop is reduced. As is shown in FIG.'s 8A and 8B, the degassing flow loop pressure can have a linear relationship with outlet CO₂ concentration. The pressure in the degassing flow loop, and in the degas vessel in particular, can be affected by the action of the fluid pump pulling fluid through the degas flow restrictor and the vacuum pump acting to remove the released gases from the degassing vessel. The action of the vacuum pump allows released gases to be vented from the degas vessel when the degas vessel is operated at pressures substantially below ambient. This, in turn, can allow for the removal of additional CO₂.

The outlet CO₂ concentration can be dependent on the inlet CO₂ concentration, the fluid pressures within the degassing flow loop, and the rates of flow through dialysate flow path and the degassing flow loop. The dialysate flow path and the degassing flow loop can operate in parallel or in series. FIG.'s 9A and 9B show the amount of CO₂ removed, and the effect on pH with differing inlet CO₂ concentrations. In all trials, the flow rates through the dialysate flow path and degassing flow loop were held at 300 mL/min and the degassing loop fluid pressure was held constant at 630 mmHg vacuum. As can be seen, the outlet CO₂ concentration is not significantly affected by large changes in the inlet CO₂ concentration. In all cases, the outlet CO₂ concentration was reduced to between 75-85 mmHg, despite the variations in inlet CO₂ concentrations.

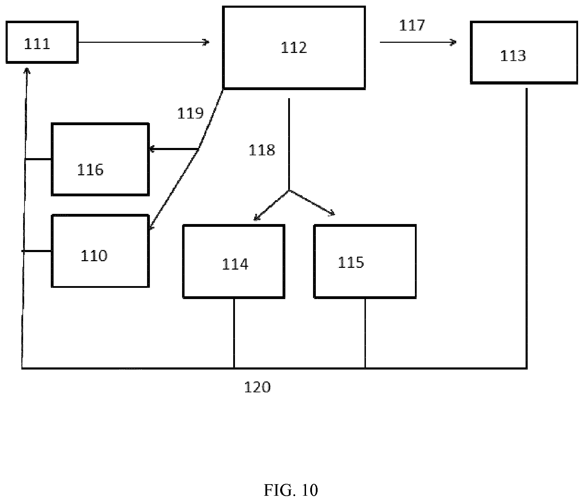

FIG. 10 shows a flow diagram, explaining one non-limiting embodiment of the operation of the vacuum pump and fluid pump of the first, second and third aspects of the invention in relation to the data received from the CO₂ sensor. In FIG. 10, both the vacuum pump and the liquid pump may be operated simultaneously. Data received from the CO₂ sensor 111 is transmitted to controller **112.** If the CO₂ concentration detected by the CO₂ sensor **111** is within the desired range in step **117,** the controller **112** can continue operating the pumps in the same manner in step **113.** If the CO₂ concentration detected by the CO₂ sensor **111** is too low **118,** the controller **112** can do either of two options. The controller **112** can cause the fluid pump to decrease the flow rate in the degassing flow loop in step **114,** causing the absolute pressure of the fluid in the degassing loop to increase and thereby reduce the amount of CO₂ removed by the degasser as shown in FIG.'s 3 and 7. Step **114** can alternatively involve that the fluid pump is shut off completely, thereby stopping the removal of CO₂ from the dialysate. Alternatively, the controller **112** can decrease the pump rate of, or shut off completely, the vacuum pump in step **115.** In certain embodiments, both steps **114** and step **115** can be carried out in response to a signal showing the CO₂ level to be too low. Decreasing the pump rate of the vacuum pump, or shutting the vacuum pump off completely, will result in less gas being removed from the degas vessel. If the CO₂ concentration detected by the CO₂ sensor **111** is too high **119,** the controller **112** can cause the fluid pump to increase the flow rate through the degassing flow loop in step **116,** and thereby increase the amount of CO₂ removed by the degasser as shown in FIG.'s 3 and 7. The controller **112** can increase the pump rate of the vacuum pump in step **110,** to remove the increased amount of gas being released from solution when the flow rate through the fluid pump is increased **116** which also enables the proper liquid level to be maintained in the degas vessel when the pressure within the degas vessel is reduced and causes the removal of more CO₂. Steps **116** and **110** can both be carried out in response to a signal showing that the CO₂ concentration is too high. Regardless of the action taken in response to the data received by the CO₂ sensor **111,** the CO₂ concentration in the dialysate can be continuously monitored, as represented by arrow **120,** and further adjustments to the rate of the fluid pump can be made as the CO₂ concentration in the dialysate changes. The vacuum pump may run continuously with the exception of step **115,** to draw out the CO₂ from the degas vessel as the CO₂ accumulates.

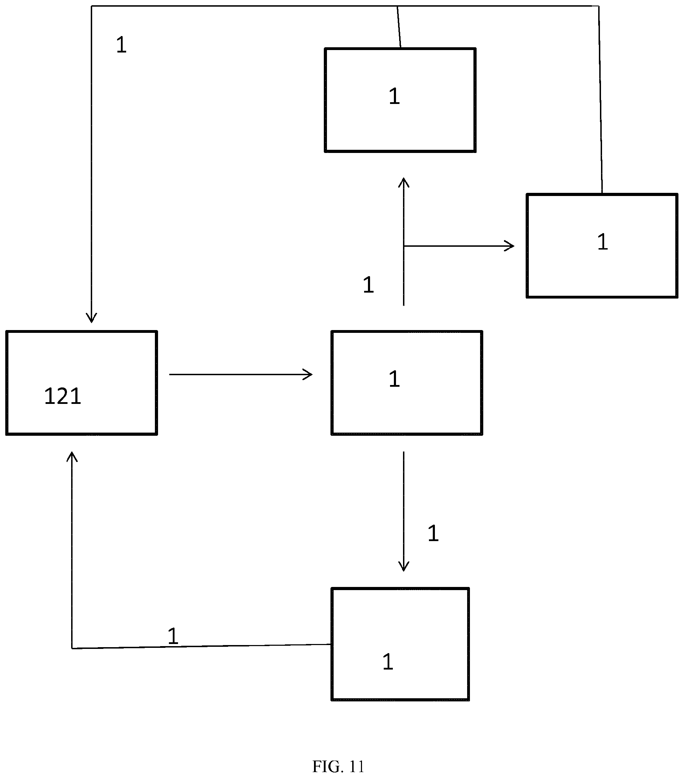

FIG. 11 shows an alternative embodiment to that shown in FIG. 10, where the vacuum pump and fluid pump are run alternately. The fluid pump can be operated to pull fluid through the degassing flow loop. Data is sent from the CO₂ sensor **121** to the controller **122** showing the CO₂ concentration in the dialysate. While the CO₂ concentration in the dialysate is above the desired range **123,** the fluid pump can be operated as explained above to remove CO₂ from the dialysate. The CO₂ concentration can be continuously monitored as the fluid pump operates, as shown by arrow **128.** Once the CO₂ concentration has decreased into the desired range **127,** the controller **112** can cause the fluid pump to shut off **124.** Simultaneously, the vacuum pump can be turned on **125** to remove the gases that have collected in the degas vessel. While the fluid pump is shut down, the CO₂ concentration in the dialysate will increase, due to the fact that dialysate is not being directed through the degasser, and will be monitored as shown by arrow **129.** When the CO₂ concentration has risen **126** to a desired range **123,** the fluid pump can again be operated and the vacuum pump shut off.

The controller can set initial pump rates for both the vacuum pump and fluid pump based on the initial carbon dioxide concentration in the dialysate. For example, if the initial carbon dioxide concentration in the dialysate is 415 mmHg partial pressure, the fluid pump and vacuum pump may be set to maintain an absolute pressure in the degas vessel of 100 mmHg. As shown in FIG. 3, this would allow for an outlet CO₂ concentration of between 50-120 mmHg partial pressure. If, during operation, the concentration of carbon dioxide were to become reduced to 117 mmHg partial pressure, the controller can alter the pump rates of the fluid pump and/or vacuum pump as described above to maintain an absolute pressure in the degas vessel of 190 mmHg. As shown in FIG. 3, this would maintain a carbon dioxide level above 50 mmHg partial pressure.

In certain embodiments, the degasser can be located in a fluid flow path in a position directly after the sorbent cartridge. The position of the degasser, however, is not limited to any one position. Alternatively, the degassing module may be located in other positions between the sorbent cartridge and the dialyzer.

To make use of the dialysis system easier, the valves and pumps may be operated by a programmable controller or computer system that can be programmed to regulate flow through the pumps and valves and into and out of the reservoirs. A rotometer or turbine with optical sensor, photocell, magnetic sensor, or other flow sensing apparatus may detect the flow of fluid through any two points in the degassing system. For example, an optical fluid flow device can be provided for measuring flow wherein the device includes an optical fluid pressure measuring device having sensors positioned in any one of the flow paths between the reservoirs, in the connectors, or in the valves or valve assemblies. The optical fluid sensors described above can be connected to an interferometer associated with an opt-electronic demodulator which has an output signal representing the differential pressure between the two sensed areas. In certain embodiments, a flow sensing apparatus can have a flow-responsive element projecting into a fluid flow path, and a position sensor associated with the element which detects a change in position of the flow-responsive element in response to the fluid flow. The flow-responsive element can be made of a wide variety of materials having the desired properties known to those of ordinary skill in the art.

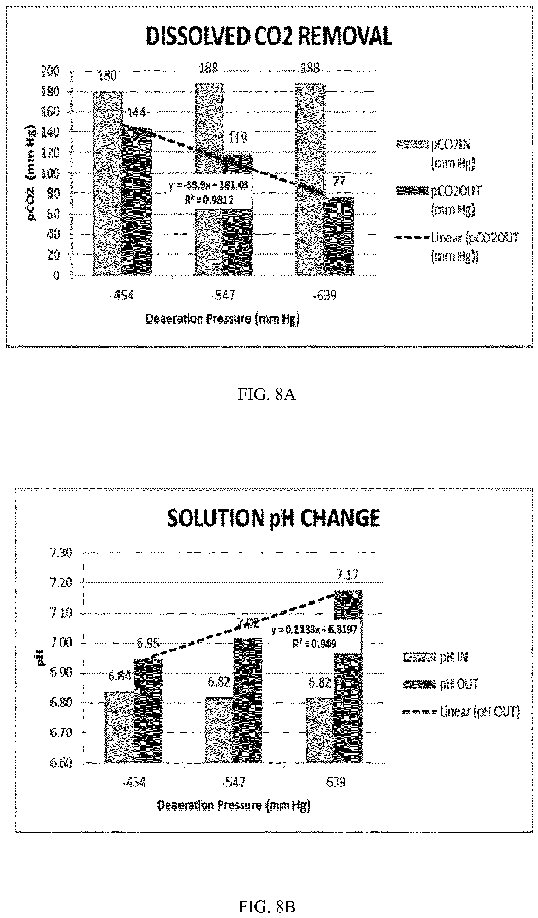

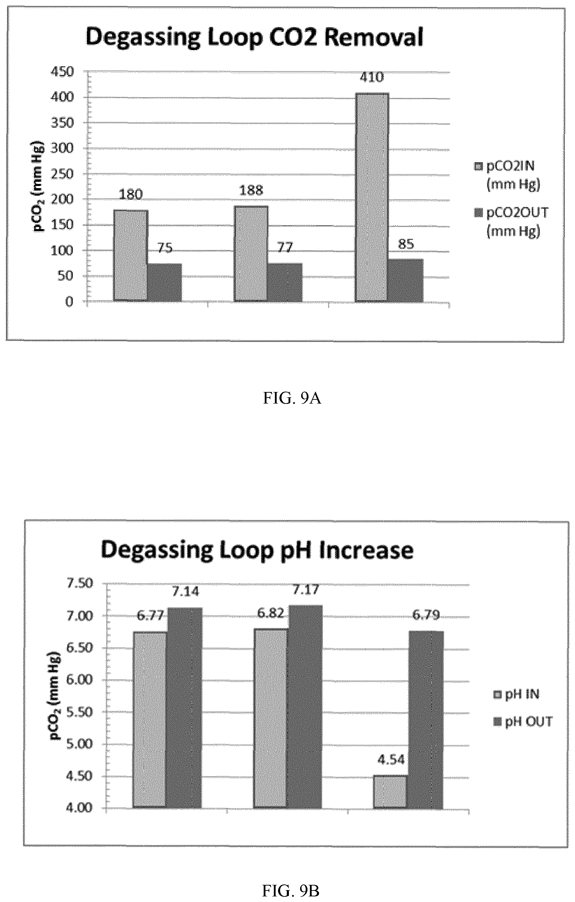

The reader is directed to FIG. 8A, which demonstrates the relationship between the pressure in the degasser and the concentration of dissolved carbon dioxide in the fluid that has passed through the degasser, and also to FIG. 9A, which demonstrates that the carbon dioxide concentration in the fluid that has passed through the degasser remained constant in a tight range when the carbon dioxide concentration in the fluid entering the degasser was more than doubled. As illustrated in FIG. 8A and FIG. 9A, the operating pressure of the degasser can be used to control the concentration of carbon dioxide in the fluid exiting the degasser.

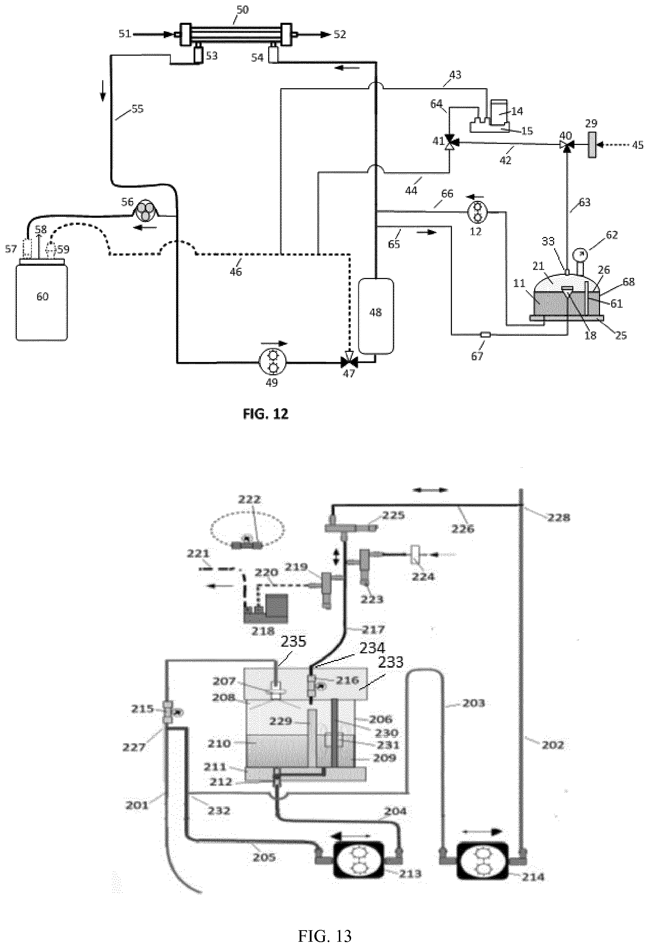

Referring to FIG. 12, a description is provided of how the concentration of dissolved carbon dioxide in the dialysate can be controlled by controlling the operating fluid pressure in the degasser to a predetermined level. Blood enters dialyzer **50** as shown by arrow **51** and exits the dialyzer **50** as shown by arrow **52.** Dialysate recirculating in dialysate flow path **55** enters the dialyzer **50** at connector **54** and exits the dialyzer **50** at connector **53** with urea that has been removed from the blood. The dialysate is pumped by dialysate pump **49** through drain valve **47** and through sorbent cartridge **48** where the urea is removed from the dialysate by an exchange process that results in carbon dioxide being added to the dialysate as the dialysate flows through sorbent cartridge **48.** The dialysate exiting the sorbent cartridge **48** is drawn into the degassing system by action of fluid pump **12** through inlet line **65.** The dialysate passes through degas flow restrictor **67** where the fluid pressure is reduced by the pressure drop that occurs as the dialysate flows through the degas flow restrictor **67.** The dialysate enters degassing vessel **68** and passes through optional degas sprayer **18** that acts to increase the surface area of the liquid and thereby increase the rate at which the dissolved carbon dioxide is released from the fluid to the gas space **21** at the top of the degassing vessel **68.** Carbon dioxide gas is collected in the gas space **21** and the degassed fluid is collected in the liquid space of degassing vessel **11.** Gas bubbles in the liquid rise to be collected in gas space **21** and the liquid exits the base **25** of degassing vessel **68** and passes through fluid pump **12** and is returned to the recirculating dialysate flow path **55** through return line **66.**

The released gas can exit the degassing vessel **68** at outlet connector **33** and pass through vent line **63** to vent control valve **40** through outflow line **42** to outflow valve **41.** During degassing, outflow valve **41** directs the flow path to gas removal pump assembly **15** through gas removal line **64.** Vacuum pump **14** pulls the gas from the low pressure environment of degassing vessel **68** and pumps the gas out through degassing outlet line **43.** Degassing outlet line **43** can optionally be connected to drain line **46.** Connecting degassing outlet line **43** to drain line **46** muffles the noise of the vacuum pump **14** and directs any condensed water vapor to reservoir **60** through drain line **46** and connector **59.** The removed gas flows out of reservoir **60** through vent **58.**

Level sensor **61** can measure the liquid level **26** in degassing vessel **68.** Level sensor **61** can be an ultrasonic sensor. Level sensor **61** can be an array of reed switches that detect the height of a magnetic float. Level sensor **61** can include a linear array of Hall-effect sensors. The rate of vacuum pump **14** can be increased to increase the liquid level **26** when level sensor **61** detects that the liquid level **26** is below a predetermined level. The rate of vacuum pump **14** can be reduced when the level sensor **61** detects that the liquid level **26** is above a predetermined level. The vacuum pump **14** can act as a check valve preventing air or liquid from returning to the degasser through degassing outlet line **43,** but can allow gas outflow from the degasser through degassing outlet line **43** including when the gas removal pump is deenergized or turned off. Air can be rapidly evacuated from the dialysate flow path **55** through outlet connector **33,** vent line **63,** vent control valve **40,** degassing outflow valve **41** and gas removal pump assembly **15** and degassing outlet line **43** during priming operations when the liquid entering the dialysate flow path **55** causes the pressure to increase, forcing the air in the gas space **21** of degassing vessel **68** through outlet connector **33** when the pressure in gas space **21** is greater than atmospheric pressure.

Vent control valve **40** can be switched to filter **29** and air can be drawn into the degassing vessel **68** as depicted by arrow **45** when liquid is being drained from the recirculating dialysate flow path **55** through drain valve **47** through drain line **46** and connector **59** to reservoir **60.** Filter **29** can have a pore size that excludes microbes and particulate to prevent contamination of the system when air is drawn in.

During flushing, cleaning and disinfection of the dialysis system, degassing vessel **68** can be completely filled with liquid and liquid can be passed out through outlet connector **33** through vent line **63,** vent control valve **40,** and degassing outflow valve **41** to recirculation line **44.** This flow path enables cleaning and disinfection solutions, including the non-limiting examples of hot water, heated citric acid solution, and bleach to be recirculate through the outlet connector **33,** vent line **63,** and vent control valve **40.** In this manner microbiological contamination and biofilms can be minimize in the degassing vessel **68** and also in the flow path used to bring air into the system when liquid is being drained from the system.

The flow restrictor **67** can have a fixed restriction, or can comprise a pressure regulator that changes the amount of flow restriction as the pumping rate of fluid pump **12** changes, such that a predetermined pressure is maintained in the dialysate exiting the restrictor across a range of operating rates of fluid pump **12.** The amount of restriction caused by flow restrictor **67** can be controlled to achieve a predetermined pressure in the fluid passing through the degasser.

Pressure sensor **62** can measure the fluid pressure in the degassing system. Pressure sensor **62** can be located on the degassing vessel **11** and can measure the pressure in the liquid or the gas. Pressure sensor **62** can be located at any point in the degasser between the flow restrictor **67** and fluid pump **12.** The pressure measurement obtained from pressure sensor **62** can be used to adjust the restriction of flow restrictor **67** to obtain a predetermined pressure in the degassing system. The rate of fluid pump **12** can be controlled to achieve a predetermined fluid pressure in the degassing system. The rate of fluid pump **12** can be increased to reduce the fluid pressure in the degasser if the fluid pressure measured by pressure sensor **62** is above the predetermined pressure. The rate of fluid pump **12** can be decreased to increase the fluid pressure in the degasser if the fluid pressure measured by pressure sensor **62** is below the predetermined fluid pressure.

In FIG. 12, an alternative control scheme is shown, wherein the pressure in the gas space **21** can be controlled by vacuum pump **14.** The pressure in the gas space **21** can be measured by pressure sensor **62** and a controller can adjust the rate of vacuum pump **14** to keep the pressure in gas space **21** at a predetermined level. In this alternative control scheme, the rate of fluid pump **12** can be increased to decrease the liquid level **26** in degassing vessel **68** or the rate of fluid pump **12** can be decreased to increase the liquid level **26** in degassing vessel **68.** In this scheme, liquid level measurements from level sensor **61** can be used to determine whether the rate of fluid pump **12** should be increased or decreased. Those of skill in the art will note that the rate of fluid pump **12** can be maintained at a constant rate while increasing the amount of flow restriction caused by flow restrictor **67** to decrease the liquid level **26** in degassing vessel **68** or decreasing the amount of flow restriction caused by flow restrictor **67** to increase liquid level **26** in degassing vessel **68.**

FIG. 13 is the degassing system of the invention for use in dialysis that reduces foaming. During treatment, dialysate is pumped from a dialyzer (not shown) through dialysate line **201.** Dialysate can enter degassing vessel **206** through a fluid inlet **235** and into degas sprayer **207,** which enters the degassing vessel **206** through a top **233** of degassing vessel **206.** In certain embodiments, a fluid inlet (not shown) can be located at the bottom of the degassing vessel **206** or at any other location relative to the degassing vessel **206.** The fluid inlet **235** can be fluidly connectable to a dialysate flow path via the dialysate line **201.** An internal conduit or passageway can convey the fluid to the degas sprayer **207** at the top **233** of the degassing vessel **206.** The top portion **208** of the degassing vessel **206** can also be referred to as the "headspace." The degas sprayer **207** sprays dialysate downwardly into the degassing vessel **206.** Foaming can be controlled by spraying downwardly onto a liquid pool in the degassing vessel **206.** The downward spray cuts the upward growth of foam, as described. The degassing vessel **206** can be separated into a spray chamber **210** and a float chamber **209** by separator **229.** A channel (not shown) can be included in separator **229** to allow fluid to move from the spray chamber **210** to the float chamber **209** to provide an accurate reading on fluid level. Any fluid connections in addition to a channel such as a passageway or other means to equilibrate the fluid level between the spray chamber **210** and a float chamber **209** is contemplated. In a preferred embodiment, the fluid connections connecting the spray chamber **210** and the float chamber **209** are located in a lower portion of the degassing vessel **206.** The float chamber **209** can include one or more level sensors. As illustrated in FIG. 13, the level sensor can include a magnetic float **231** on guide **230.** A linear array of Hall effect sensors (not shown) can be included to measure the level of the float **231** and determine the fluid level in the degassing vessel **206.** In certain embodiments, the float **231** can be magnetic, and a linear array of Hall effect sensors can measure the height of the float directly. Alternatively, a magnet can be affixed to the float **231.** In alternative embodiments, the level sensors can include a capacitive or ultrasonic sensor to measure the height of the liquid directly. An ultrasonic sensor emits an ultrasonic wave and measures the distance to the liquid based on the time between emission of the wave and detection of the wave reflected back by the liquid. A capacitive sensor measures distance to the liquid by measuring changes in capacitance as the liquid moves closer to or further away from the sensor. The height of the fluid in the degassing vessel **206** can be controlled to within a predetermined range to ensure that the liquid level is below the degas sprayer **207,** ensuring that the degas sprayer **207** nozzle is exposed and that atomized fluid is exposed to the low pressure in the top portion **208** of the degassing vessel **206.** The fluid level should also remain low enough that loss of liquid through the gas outlet line **217** is prevented, and high enough such that undissolved gas bubbles in the liquid are separated and captured.

Fluid can be sprayed into the spray chamber **210** of the degassing vessel **206.** Gas can be removed from the fluid through a gas outlet **234** fluidly connectable to gas outlet line **217.** A gauge pressure sensor **216** in the gas outlet **234** can measure the pressure inside the degassing vessel **206.** Gas bubble nucleation can occur as the fluid is sprayed into the spray chamber **210.** Before the gas bubbles can exit the degassing vessel **206,** the gas bubbles rise through the liquid and are captured and collected in a headspace of the degassing vessel **206.** Bubble capture can be ensured when the downward velocity of the liquid in the degassing vessel **206** is less than the rise velocity of the bubbles through the liquid. The degas sprayer **207** atomizes the fluid and creates a high surface area to volume ratio between the liquid droplets and gas in the degas vessel headspace. A vacuum pump **218** is used to lower the pressure in the degassing vessel **206,** and is fluidly connectable to gas outlet line **217** by valve **219** and vacuum line **220** and can be controlled by a controller to maintain a desired pressure within the degassing vessel **206.** In a preferred embodiment, the vacuum pump **218** is continuously run at a high rate, and the controller can pulse width modulate valve **219** to control the pressure in the degassing vessel **206** to a desired target. The removed gases are expelled through gas line **221,** which can be vented to the air, or alternatively, connected to a waste reservoir.

In a preferred embodiment, the vacuum pump **218** is continuously run at a high rate. Valve **219** is closed when the gas outlet pressure drops below a target pressure and opened when the gas outlet pressure rises above the target pressure. Controlling pressure by only modulating valve **219** maintains the atmosphere within the top portion **208** of the degassing vessel **206** as predominately CO₂. Opening vent valve **223** would allow air into the degassing vessel **206,** changing the ratio of carbon dioxide, nitrogen, and oxygen in the degassing vessel **206** making control over carbon dioxide difficult. By maintaining a predominately carbon dioxide atmosphere in the degassing vessel **206,** the system can precisely control the amount of dissolved carbon dioxide in the dialysate.

Degassed fluid can exit the degassing vessel **206** through a liquid outlet **212** in a base 211 of the degassing vessel **206,** fluidly connectable to fluid line **204.** The liquid outlet **212** is located at a lower elevation in the degassing vessel **206** than the gas outlet **234** at gas outlet line **217.** Fluid can be pumped by fluid pump **213,** through fluid line **205,** and back to dialysate line **201** at junction **227.** The fluid pump **213** provides the force necessary to move fluid from the low pressure degassing vessel **206** to the higher pressure in dialysate line **201.** The fluid lines **204, 205,** and **201,** with degassing vessel **206,** form a degassing flow path that is parallel to a main dialysate flow path. Fluid can be pumped from the degassing flow path at junction **232** into the main dialysate flow path through fluid line **203** by dialysate pump **214** into dialysate line **202.** The flow rate of fluid through the main dialysate flow path can be controlled by dialysate pump **214,** and optionally one or more additional dialysate pumps. As such, the flow rate of fluid through the degassing flow loop can be controlled independently of the flow rate of fluid in the main dialysate flow path. By operating fluid pump **213** at a higher pump rate than dialysate pump **214,** fluid can be recirculated through the degassing vessel **206** multiple times prior to returning to the main dialysate flow path, allowing additional control over the amount of gas removed. The rate of liquid recirculation through the degassing vessel **206** can help to ensure sufficient exposure to the headspace of the degassing vessel **206** so that dissolved gases in the liquid come into equilibrium with the gas partial pressures in the degassing vessel **206.** In certain embodiments, the flow rate of fluid through the degassing flow loop can be set to about two times the dialysate flow rate. The fluid pump **213** and dialysate pump **214** can be controlled by a controller (not shown) to operate at the desired ratio.

The fluid inlet **235,** gas outlet **233,** and liquid outlet **212** are each fluidly connectable to the dialysate flow path. In certain embodiments, the fluid inlet **235,** gas outlet **233,** and liquid outlet **212** can be disconnected from the dialysate flow path to allow the degassing vessel **206** to be removed from the system for maintenance or replacement. The degassing vessel **206** can be reconnected to the system by connecting fluid inlet **235,** gas outlet **233,** and liquid outlet **212** to lines **201, 217,** and **204,** respectively. Any method known in the art can be used to fluidly connect fluid inlet **235,** gas outlet **233,** and liquid outlet **212** to the dialysate flow path, including quick-connect fittings, screw fittings, or any other method or mechanical fastening means.

A vent valve **223** fluidly connected to the gas outlet line **217** can be controlled to allow air into the degassing vessel **206** when the degassing vessel **206** is drained. Filter **224** prevents contamination of the degassing vessel **206,** and can have a pore size that excludes microbes and particulate matter to prevent contamination of the system when air is drawn in through vent valve **223.** During flushing, cleaning and disinfection of the dialysis system, degassing vessel **206** can be completely filled with liquid and liquid can be passed out through gas outlet line **217** through valve **225** and fluid line **226,** to dialysate line **202** at junction **228.** The flow path enables cleaning and disinfection solutions, including the non-limiting examples of hot water, heated citric acid solution, and bleach to be recirculate through all of the lines of the degassing system. In this manner, microbiological contamination and biofilms can be minimized in the degassing vessel **206** and also in the flow path used to bring air into the system when liquid is being drained from the system. A temperature sensor (not shown) can be included to monitor the temperature during disinfection, and to measure the temperature of dialysate prior to reaching a heater (not shown) in the dialysate flow path. An ambient pressure sensor **222** can measure the atmospheric pressure outside of the degassing system, and is used conjunction with gauge pressure sensor **216** to determine the absolute pressure in the headspace, or top portion **208** of the degassing vessel **206.**

During treatment, the degassing system should control carbon dioxide removal to maintain a carbon dioxide level within a desired range. In certain embodiments, the desired range can be from 40mmHg - 150mmHg pCO₂. The concentration of the dissolved gases in the dialysate exiting the degassing vessel **206** are proportional to the absolute partial pressures of the gas in the top portion **208,** and as such, the environmental pressure as measured by ambient pressure sensor **222** can be used to control the gas pressure within the degassing vessel **206.** Ambient pressure sensor **222** measures the absolute pressure of the environment outside of the degassing vessel **206.** Gauge pressure sensor **216** measures a gauge pressure referenced to the ambient pressure sensor **222.** The pressure as measured by ambient pressure sensor **222** plus the gauge pressure measured by gauge pressure sensor **216** provides the absolute pressure in the top portion **208** of the degassing vessel **206.** Alternatively, the gauge pressure sensor **216** can be replaced by an absolute pressure sensor to measure the absolute pressure in the headspace, or top portion **208** of the degassing vessel **206,** and the ambient pressure sensor **222** is not required. The dialysate flow rate also controls the amount of gas removed. In certain embodiments, the dialysate flow rate through the degassing flow loop can be from 100mL/min to 800mL/min. In certain embodiments, the dialysate flow path can include a heater (not shown) to heat the dialysate to a desired temperature prior to reaching the dialyzer. The degassing flow loop can be positioned either upstream or downstream of the heater. The degassing system should be able to operate over the entire possible range of dialysate temperatures. When positioned downstream of the heater, the dialysate temperature in the degassing flow loop should be from about 35°C to about 39.5°C. When positioned upstream of the heater, the possible temperature range of dialysate in the dialysate flow path can be larger, including from between about 10°C to about 45°C.

The amount of gas removed by the degassing system is a function of the absolute headspace pressure in the degassing vessel **206,** as well as the degassing flow loop flow rate. In some embodiments, the headspace pressure of the degassing vessel **206,** an estimated degasser inlet carbon dioxide concentration is used, as described. In a preferred embodiment, the size and flow rate through the degassing flow loop and degas sprayer **207** is sufficient to ensure that dissolved gases in the liquid exiting the degassing vessel **206** through fluid line **204** are in approximate equilibrium with the gas partial pressure in the top portion **208,** or headspace, of the degassing vessel **206.** When the dissolved gases in the liquid are in approximate equilibrium with the gas partial pressure in the top portion **208** of the degassing vessel **206,** the carbon dioxide pressure can be controlled by controlling the absolute headspace pressure. As such, the carbon dioxide pressure (dissolved CO₂ concentration) in the degassed dialysate flowing to the dialyzer can be controlled across a very wide range of inlet carbon dioxide pressures. The headspace pressure can be controlled to a predetermined target, irrespective of the estimated carbon dioxide concentration in the liquid entering the degassing vessel through dialysate line **201.** In certain embodiments, the vacuum pump **218** is operated by the controller at a fixed rate. The absolute headspace pressure in the degassing vessel **206** is equal to the degassing vessel pressure as measured by gauge pressure sensor **216** plus the atmospheric pressure as measured by absolute ambient pressure sensor **222.** Valves **219** and **223** can be selectively controlled by the controller to allow the vacuum pump **218** to remove air from the degassing vessel **206** or to allow air to flow into degassing vessel **206,** thereby controlling the headspace pressure to the headspace pressure set point. In certain embodiments, the estimated degasser inlet carbon dioxide concentration can vary as a profile during a dialysis session, and as such, the headspace pressure set point can also vary during treatment. The degassing flow loop flow rate can be controlled by using a fixed pressure change to achieve a desired flow rate. The pressure change can be measured by the difference between the incoming fluid pressure as measured by pressure sensor **215** and the pressure within the degassing vessel **206** measured by gauge pressure sensor **216.** Using the fixed pressure change, a pressure change set point can be set, and the fluid pressure at pressure sensor **215** varied by changing the fluid pump **213** rate until the pressure change set point is reached. In certain embodiments, the relationship between the pressure change and the flow rate can be empirically determined. Alternatively, the relationship can be calculated using an algorithm. The degassing flow loop flow rate should be set at a rate sufficient to ensure the dialysate comes into approximate equilibrium with the gas pressures in the degassing vessel **206,** but low enough to avoid over degassing, erratic level behavior, or excess foam generation. In certain embodiments, the degassing flow loop flow rate can be set between 750 and 800 mL/min. Over degassing with a degassing flow loop flow rate of ∼800 mL/min and a dialysate flow rate of about 100 mL/min has not been observed. If the pump rate of fluid pump **213** deviates from the normal relationship with the pressure change, an obstruction in the fluid inlet **235** of the degassing vessel **206** or an error in the control over the pressure change may be indicated.

If an error is indicated, the system can generate an alert informing the user of the error and/or stop treatment. In certain embodiments, a protective system can be used. The protective system can receive the dialysate flow rate from a flow sensor (not shown) in the dialysate flow path and determine the change in pressure set point to operate the degassing flow loop flow rate at a set ratio to the dialysate flow path flow rate. The protective system can determine an expected operating rate (RPM) of the fluid pump **213** corresponding to the pressure change set point, and calculate a running average operating rate for fluid pump **213.** The protective system can generate an alert if the running average of RPM for fluid pump **213** is outside of a predetermined range of the expected value. In certain embodiments, the predetermined range can be ±10% of the expected value. The protective system can also monitor the pressure in the headspace of the degassing vessel **206.** The protective system can measure the ambient pressure with ambient pressure sensor **222** and the pressure inside the degassing vessel **206** with gauge pressure sensor **216** to calculate the absolute pressure within the degassing vessel **206** and can calculate a running average of the absolute pressure. The running average of absolute pressure can be compared to a predetermined limit, and an alert generated if the absolute pressure is outside of the predetermined limit.

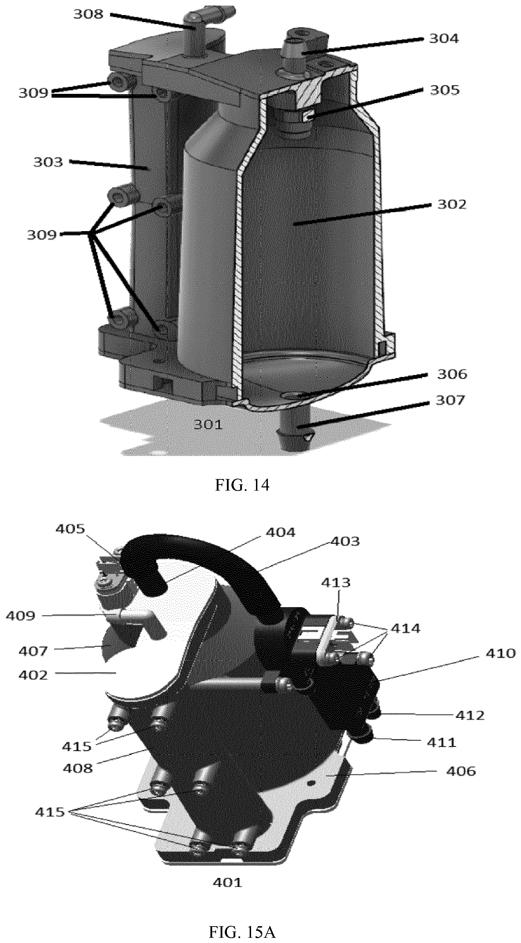

FIG. 14 shows a cross section of a degassing vessel **301** for use in dialysis. The degassing vessel **301** can be divided into a spray chamber **302** and a float chamber **303.** As described, the float chamber **303** can contain one or more level sensors in communication with a controller. In certain embodiments, the level sensors can be a magnetic float and a linear array of Hall effect sensors (not shown). Fluid enters the degassing vessel **301** through a fluid inlet **304** fluidly connected to a degas sprayer nozzle **305.** The degas sprayer nozzle **305** sprays the fluid into the spray chamber **302.** One or more channels (not shown) connect the spray chamber **302** to the float chamber **303** equilibrating the fluid level in each chamber. Degassed fluid exits the degassing vessel **301** through opening **306** fluidly connected to liquid outlet **307.** In certain embodiments, the liquid outlet **307** is located in a bottom portion of the spray chamber **302.** By placing the liquid outlet **307** at the bottom portion of the spray chamber **302,** fluid may enter or exit the float chamber **303** only when the fluid level in the degassing vessel **301** is changing, reducing turbulence in the float chamber **303** and reducing the amount of gas bubbles that come out of solution in the float chamber **303.** By reducing turbulence and gas bubbles in the float chamber **303,** a more accurate and stable detection of fluid level may be achieved. Alternatively, the liquid outlet **307** can be positioned in a bottom portion of the float chamber **303** or between the spray chamber **302** and float chamber **303.** Further, placing the liquid outlet **307** at the bottom portion of the spray chamber **302** increases the recirculating flow rate of fluid in the degassing flow loop, which beneficially increases the gas removal rate.

Gases can be removed from the degassing vessel **301** through gas outlet **308,** which can be fluidly connected to a vacuum pump (not shown) by one or more valves. In a preferred embodiment, the gas outlet **308** is positioned at a top portion of the degassing vessel **301** between the spray chamber **302** and the float chamber **303.** Placing the gas outlet **308** between the spray chamber **302** and float chamber **303** allows symmetrical gas removal from both chambers to maintain equal pressures in both chambers while preserving the filling, draining, and disinfection capabilities of the degassing vessel **301.** Holes **309** can be included for securing a circuit board including the linear array of Hall effect sensors to detect the level of the float (not shown) and therefore the liquid level in the degassing vessel **301.**

As illustrated in FIG. 14, the spray chamber **302** can have a substantially conical shape, as opposed to a tubular or other shape. The conical shape of the spray chamber **302** can cause spraying fluid to contact the walls of the spray chamber **302** at a shallower angle than if the spray chamber **302** has a tubular shape. The shallow angle of spray contacting the walls of the spray chamber **302** may result in less turbulence, reducing foaming of the fluid in the degassing vessel **301** and allowing more accurate measurements of the fluid level. The conical film of spray existing the degas sprayer nozzle **305** and impinging on the cone wall creates a foam cutting barrier above which the foam cannot grow. Reducing foaming also reduces gas flow restrictions out of the degassing vessel **301,** allowing for a higher gas removal volume and faster headspace vacuum pressure recovery. When foam exits thru gas outlet **308** the foam can impede the gas flow thru the vacuum pump, causing an abrupt increase in headspace pressure. Preventing the foam from rising to gas outlet **308** prevents the gas flow from the vacuum pump from being restricted by the foam and the headspace can more quickly recover from any perturbation.

The spray chamber **302** can be any length and diameter sufficient to effectively capture bubbles in the fluid sprayed into the spray chamber **302.** In certain embodiments, the spray chamber **302** can have a diameter of about 75 mm and a height of about 10 cm, which gives a balance of degassing capacity and foam control without excessive size or fluid volume. In other embodiments, the diameter can be from about 50 mm to about 100 mm, including between 50 mm and 75 mm, between 50 mm and 60 mm, between 60 mm and 100 mm, or between 75 mm and 100 mm. The height of the spray chamber **302** can be from about 60 mm and about 200 mm, including between 60 mm and 100 mm, between 60 mm and 75 mm, between 70 mm and 100 mm, between 90 mm and 125 mm, between 100 mm and 150 mm, between 125 mm and 200 mm or between 150 mm and 200 mm. A larger length and diameter of the spray chamber **302** can further reduce foaming by creating a better transition zone when fluid is sprayed into the spray chamber **302.** A larger diameter spray chamber **302** also increases the surface area of the fluid and causes the sprayed liquid to have a greater contact time with the headspace, allowing more efficient gas removal.

In certain embodiments, the degas sprayer nozzle **305** can be constructed to create an even cone shaped spray, rather than a more coarse "fountain like" spray, which can further reduce foaming in the spray chamber **302.** Importantly, by placing the degas sprayer at a top of the degassing vessel **301** rather than at a base of the degassing vessel **301,** the sprayer can reduce foaming by acting as a cap to control the foam. A finer spray cone, rather than a fountain type spray, can also increase atomization of the fluid and accelerate gas removal, increasing the efficiency of the degasser.

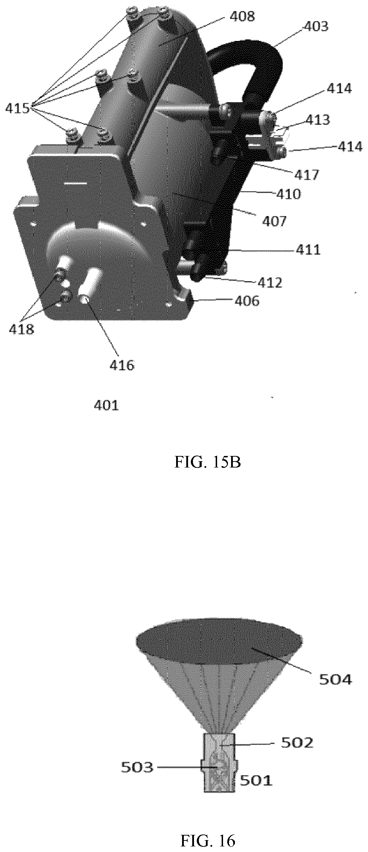

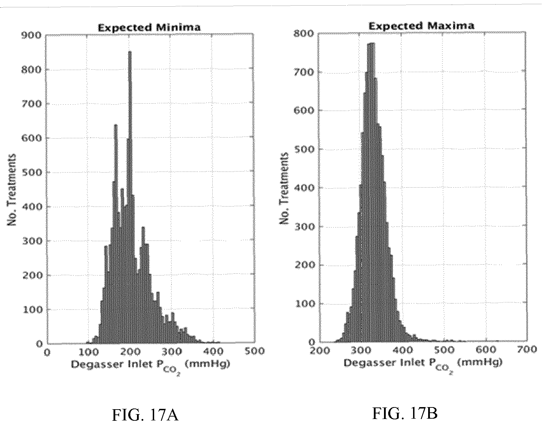

FIG. 15A is a top view of a degassing vessel **401** for use in dialysis and FIG. 15B is a side view of the degassing vessel **401.** The degassing vessel **401** can include a spray chamber **407** and a float chamber **408.** One or more level sensors (not shown) can be included in float chamber **408** to measure a fluid level in the degassing vessel **401.** In certain embodiments, a manifold **410** can house fluid flow paths in a degassing flow loop. Fluid enters the manifold **410** from a dialysate flow path (not shown) through inlet **411.** The fluid flows through fluid line **403** and fluid inlet **404** in a top portion **402** of the degassing vessel **401.** The fluid inlet **404** can be fluidly connected to a degas sprayer (not shown in FIG.'s 15A-B) in an interior of the spray chamber **407.** Gases are removed via gas outlet **409,** as illustrated in FIG. 15A, which can be fluidly connected to a vacuum pump (not shown). As illustrated in FIG. 15B, fluid exits the degassing vessel **401** through a liquid outlet **416** in a base **406** of the degassing vessel **401.** Fluid lines (not shown) can connect the liquid outlet **416** to a fluid pump (not shown), and back to the manifold **410** through a second inlet **412** to recirculate the fluid in the degassing flow loop. Fluid can be directed back to a dialysate flow path, parallel to the degassing flow loop, through outlet **417** in manifold **410.** Gauge pressure sensor **405** can measure the pressure in the headspace of the degassing vessel **401.** Pressure sensor **413** can measure the pressure of the incoming liquid, which may be used to control the pump rates of the fluid pumps. Mounting bases **414, 415,** and **418** can be included to attach the circuit board including the linear array of Hall effect sensors to detect the float in the float chamber **408.**

As described, a cone-like spray from the degas sprayer reduces foaming in the degassing vessel. FIG. 16 illustrates a non-limiting embodiment of a spray nozzle **501** for use in a degassing system. The spray nozzle **501** includes an internal conduit **502** through which fluid flows. The internal conduit **502** can include one or more swirl inducing sets **503,** which force the fluid into a vortex motion within the internal conduit **502,** breaking the fluid apart. As a result, fluid exiting the spray nozzle **501** produces a full cone **504** that evenly distributes the spray pattern. The high surface area to volume ratio in the cone **504** allows gas to rapidly move from solution into equilibrium with the low gas pressure inside the degassing vessel. The spray nozzle **501** used with a degasser influences the relationship between the flow rate and the pressure change from the fluid inlet to the inside of the degassing vessel. As described, the relationship between the flow rate and pressure change can be used to control the degassing flow loop flow rate, and the pressure change set point can be adjusted based on the spray nozzle used. In a preferred embodiment, the spray is a dense conical film that smashes foam bubbles as the foam head grows upward, thus limiting the upward growth of the foam to the surface height defined by the spray cone.

The degassing system should be able to control the carbon dioxide concentration in the dialysate flow path at the dialyzer inlet to a specified range, which in certain embodiments can be from 40mmHg - 150mmHg pCO₂. The expected range of CO₂ concentrations at the inlet to the degassing flow loop can vary from between 85 to 650 mmHg pCO₂. To predict the expected degasser inlet CO₂ concentration, 10,000 simulated treatments were conducted. FIG.'s 17A and 17B illustrate the expected pCO₂ minima and maxima, respectively. A summary of the simulations are shown in Table 1.

**Table 1**

| **Expected Sorbent Outlet PCO2 Level from Model** | | | |
|---|---|---|---|
| **Scenario** | **Minimum (mmHg)** | **Maximum (mmHg)** | **Range (mmHg)** |
| **99^{th} Percentile** | **130** | **415** | **130 - 415** |
| **99.9^{th} Percentile** | **110** | **510** | **110 - 510** |
| **99.99^{th} Percentile** | **95** | **590** | **95 - 590** |
| **99.99^{th} Percentile with Engineering Margin of 10%** | **85** | **650** | **85 - 650** |

As illustrated in FIG.'s 17A-B and Table 1, the 99^{th} percentile for the minimum range for carbon dioxide concentration in dialysate exiting a sorbent cartridge, or the minimum range for carbon dioxide concentration in dialysate entering the degassing system is 130 mmHg. The 99.99^{th} percentile for the minimum range for carbon dioxide concentration is 85 mmHg, even with a built-in engineering margin of 10%. The 99^{th} percentile for the maximum range for carbon dioxide concentration is 415 mmHg. The 99.99^{th} percentile for the maximum range for carbon dioxide concentration is 650 mmHg, even with a built-in engineering margin of 10%.

Fluid entering the degassing system will also contain dissolved nitrogen and oxygen gases. Table 2 summarizes the results of simulated treatments to determine the expected concentration ranges of oxygen and nitrogen when exiting the degasser as a function of the blood flow rate QB, the dialysate flow rate QD, the type of blood access, the dialyzer used, the dialyzer mass transfer-are coefficient of the dialyzer KoA, the initial patient nitrogen and oxygen blood concentrations CBin and the degasser inlet concentrations for both nitrogen and oxygen CDin. KoA is used as an approximation for the mass transfer coefficients of oxygen and nitrogen in the calculation to estimate the O₂ and N₂ concentrations in the dialysate exiting the dialyzer and returning to the degasser. The higher the KoA for the dialyzer, the higher the capability of the dialyzer membrane to transport molecular entities across the dialyzer membrane. The simulations provided the dialysance D, as well as the degasser outlet concentration for oxygen and nitrogen C_{Dout}. The data in Table 2 was obtained assuming that the concentration of nitrogen in the patient's blood was approximately equal to atmospheric nitrogen concentration, or 600 mmHg. A low blood oxygen concentration was assumed to be 30 mmHg, while a high blood oxygen concentration was assumed to be 100 mmHg. The dialysance of oxygen and nitrogen was approximated by the KoA for urea. Table 3 summarizes the findings for possible ranges of each gas in the dialysate based on high or low values for each gas.

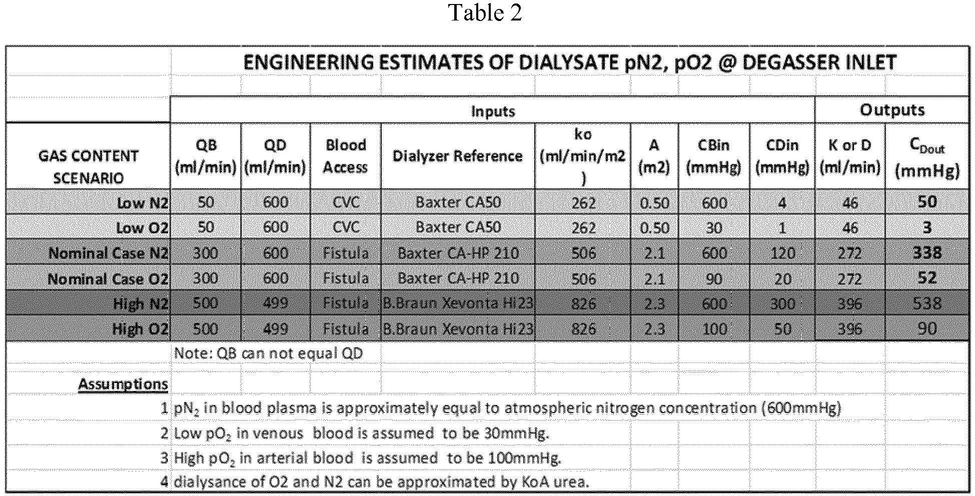

**Table 3**

| **Sorbent Outlet (Degasser Inlet) Gas Concentration Summary** | | | | | |
|---|---|---|---|---|---|
| **TEST CASE** | **pO2 (mmHg)** | **pN2 (mmHg)** | **pCO2 (mmHg)** | **%CO2** | **Test Gas Mix** |
| Low N2, Low O2, Low CO2 | 3 | 50 | 85 | 62% | 60% |
| Low N2, Low O2, High CO2 | 3 | 50 | 650 | 92% | 90% |
| Nominal N2, O2, CO2 | 52 | 338 | 300 | 43% | 40% |
| High N2, High O2, Low CO2 | 100 | 600 | 85 | 11% | 10% |
| High N2, High O2, High CO2 | 100 | 600 | 650 | 48% | 50% |

Table 3 summarizes the findings for possible ranges of each gas in the dialysate based on high or low values for each gas. As described, the possible ranges for carbon dioxide, nitrogen, and oxygen concentrations in the dialysate are used to control the degasser by setting a headspace pressure set point and degassing loop flow rate.

## Claims

1. A system for use in dialysis; comprising:
a degassing vessel (206);
a fluid inlet (235) in the degassing vessel (206) fluidly connected to a dialysate flow path;
a liquid outlet (212) in the degassing vessel (206) fluidly connected to the dialysate flow path;
a gas outlet (234) fluidly connected to a vacuum pump (218); and
a degas sprayer (207) fluidly connected to the fluid inlet (235) wherein the degas sprayer (207) is configured to direct liquid downwardly into the degassing vessel (207), wherein the degassing vessel (206) comprises a spray chamber (210) comprising a portion into which fluid can be sprayed, and a float chamber (209) comprising a fluid level sensor; the spray chamber (210) fluidly connected to the float chamber (209); wherein the degas sprayer (207) is positioned above the spray chamber (210), wherein the liquid outlet (212) is positioned in a bottom portion (211) of the spray chamber (210),
wherein a fluid line (204) fluidly connects the liquid outlet (212) to another fluid line (205) fluidly connected to the fluid inlet (235), the first of the fluid lines (204) comprising a fluid pump (213); and
further comprising a controller controlling the vacuum pump (218) and the fluid pump (213).

2. The system of claim 1, wherein the spray chamber (210) has a substantially conical shape.

3. The system of claim 1 to 2, wherein the gas outlet (234) is positioned between the spray chamber (210) and the float chamber (209).

4. The system of any of claims 1 to 3, wherein the fluid level sensor comprises one or more of: a float with a magnet (230, 231) and a linear array of Hall effect sensors, an ultrasonic sensor, and a capacitive sensor.

5. The system of any preceding claim, further comprising a temperature sensor in the liquid outlet (212), or further comprising a pressure sensor (216) in the gas outlet (234), or comprising both of these sensors.

6. The system of any preceding claim, further comprising a valve (219) positioned between the gas outlet (234) and the vacuum pump (218) or further comprising a vent valve (223) positioned between a vent and the gas outlet (234), or comprising both the valve (219) and vent valve (223).

7. The system of any preceding claim, further comprising a pressure sensor (215) upstream of the fluid inlet (235) and another pressure sensor (216) in the gas outlet (234).

8. The system of claim 7, wherein the degassing flow rate is controlled using a fixed pressure change to achieve a desired flow rate.

9. The system of any preceding claim, further comprising an ambient pressure sensor (222).

10. The system of claim 9, wherein the system is configured such that the carbon dioxide level in dialysate exiting the degassing vessel (206) is maintained at between 5333Pa and 19998 Pa (40 mm Hg and 150 mm Hg pCO₂).

## Patentansprüche

1. System zur Verwendung bei einer Dialyse; das Folgendes umfasst:
einen Entgasungsbehälter (206);
einen Fluideinlass (235) in dem Entgasungsbehälter (206), der mit einem Dialysatdurchflussweg fluidisch verbunden ist;
einen Flüssigkeitsauslass (212) in dem Entgasungsbehälter (206), der mit dem Dialysatdurchflussweg fluidisch verbunden ist;
einen Gasauslass (234), der mit einer Vakuumpumpe (218) fluidisch verbunden ist; und
einen Entgasungssprüher (207), der mit dem Fluideinlass (235) fluidisch verbunden ist,
wobei der Entgasungssprüher (207) konfiguriert ist, um Flüssigkeit nach unten in den Entgasungsbehälter (207) zu leiten, wobei der Entgasungsbehälter (206) eine Sprühkammer (210), die einen Abschnitt umfasst, in den Fluid gesprüht werden kann, und
eine Schwimmerkammer (209) umfasst, die einen Fluidfüllstandssensor umfasst; wobei die Sprühkammer (210) mit der Schwimmerkammer (209) fluidisch verbunden ist; wobei der Entgasungssprüher (207) über der Sprühkammer (210) positioniert ist, wobei der Flüssigkeitsauslass (212) in einem Bodenabschnitt (211) der Sprühkammer (210) positioniert ist,
wobei eine Fluidleitung (204) den Flüssigkeitsauslass (212) mit einer anderen Fluidleitung (205) fluidisch verbindet, die mit dem Fluideinlass (235) fluidisch verbunden ist, wobei die erste der Fluidleitungen (204) eine Fluidpumpe (213) umfasst; und
ferner eine Steuervorrichtung umfasst, die die Vakuumpumpe (218) und die Fluidpumpe (213) steuert.

2. System nach Anspruch 1, wobei die Sprühkammer (210) eine im Wesentlichen konische Form aufweist.

3. System nach Anspruch 1 bis 2, wobei der Gasauslass (234) zwischen der Sprühkammer (210) und der Schwimmerkammer (209) positioniert ist.

4. System nach einem der Ansprüche 1 bis 3, wobei der Fluidfüllstandssensor Folgendes umfasst: einen Schwimmer mit einem Magneten (230, 231) und einer linearen Anordnung von Hall-Sensoren, einen Ultraschallsensor und/oder einen kapazitiven Sensor.

5. System nach einem der vorhergehenden Ansprüche, das ferner einen Temperatursensor in dem Flüssigkeitsauslass (212) umfasst oder ferner einen Drucksensor (216) in dem Gasauslass (234) umfasst oder beide dieser Sensoren umfasst.

6. System nach einem der vorhergehenden Ansprüche, das ferner ein Ventil (219) umfasst, das zwischen dem Gasauslass (234) und der Vakuumpumpe (218) positioniert ist, oder ferner ein Entlüftungsventil (223) umfasst, das zwischen einer Entlüftung und dem Gasauslass (234) positioniert ist; oder sowohl das Ventil (219) als auch das Entlüftungsventil (223) umfasst.

7. System nach einem der vorhergehenden Ansprüche, das ferner einen Drucksensor (215) stromaufwärts des Fluideinlasses (235) und einen anderen Drucksensor (216) in dem Gasauslass (234) umfasst.

8. System nach Anspruch 7, wobei die Entgasungsdurchflussrate unter Verwendung einer festen Druckänderung gesteuert wird, um eine gewünschte Durchflussrate zu erreichen.

9. System nach einem der vorhergehenden Ansprüche, das ferner einen Umgebungsdrucksensor (222) umfasst.

10. System nach Anspruch 9, wobei das System derart konfiguriert ist, dass der Kohlendioxidfüllstand in einem Dialysat, das aus dem Entgasungsbehälter (206) austritt, zwischen 5333 Pa und 19998 Pa (40 mmHg und 150 mmHg pCO₂) gehalten wird.

## Revendications

1. Système destiné à être utilisé dans une dialyse ; comprenant :
un récipient de dégazage (206) ;
une entrée de fluide (235) dans le récipient de dégazage (206) raccordée de manière fluidique à un chemin d'écoulement de dialysat ;
une sortie de liquide (212) dans le récipient de dégazage (206) raccordée de manière fluidique au chemin d'écoulement de dialysat ;
une sortie de gaz (234) raccordée de manière fluidique à une pompe à vide (218) ; et
un pulvérisateur de dégazage (207) raccordé de manière fluidique à l'entrée de fluide (235), le pulvérisateur de dégazage (207) étant conçu pour diriger le liquide vers le bas dans le récipient de dégazage (207), le récipient de dégazage (206) comprenant une chambre de pulvérisation (210) comprenant une partie dans laquelle du fluide peut être pulvérisé, et une chambre à flotteur (209) comprenant un capteur de niveau de fluide ; la chambre de pulvérisation (210) étant raccordée de manière fluidique à la chambre à flotteur (209) ; le pulvérisateur de dégazage (207) étant positionné au-dessus de la chambre de pulvérisation (210), la sortie de liquide (212) étant positionnée dans une partie inférieure (211) de la chambre de pulvérisation (210),
une conduite de fluide (204) raccordant de manière fluidique la sortie de liquide (212) à une autre conduite de fluide (205) raccordée de manière fluidique à l'entrée de fluide (235), la première des conduites de fluide (204) comprenant une pompe à fluide (213) ; et
comprenant en outre un dispositif de régulation régulant la pompe à vide (218) et la pompe à fluide (213).

2. Système selon la revendication 1, la chambre de pulvérisation (210) ayant une forme sensiblement conique.

3. Système selon les revendications 1 à 2, la sortie de gaz (234) étant positionnée entre la chambre de pulvérisation (210) et la chambre à flotteur (209).

4. Système selon l'une quelconque des revendications 1 à 3, le capteur de niveau de fluide comprenant : un flotteur doté d'un aimant (230, 231) et d'un réseau linéaire de capteurs à effet Hall, et/ou un capteur à ultrasons et/ou un capteur capacitif.

5. Système selon l'une quelconque des revendications précédentes, comprenant en outre un capteur de température dans la sortie de liquide (212), ou comprenant en outre un capteur de pression (216) dans la sortie de gaz (234), ou comprenant à la fois ces deux capteurs.

6. Système selon l'une quelconque des revendications précédentes, comprenant en outre une valve (219) positionnée entre la sortie de gaz (234) et la pompe à vide (218) ou comprenant en outre une valve d'évent (223) positionnée entre un évent et la sortie de gaz (234), ou comprenant à la fois la valve (219) et la valve d'évent (223).

7. Système selon l'une quelconque des revendications précédentes, comprenant en outre un capteur de pression (215) en amont de l'entrée de fluide (235) et un autre capteur de pression (216) dans la sortie de gaz (234).

8. Système selon la revendication 7, le débit de dégazage étant régulé à l'aide d'un changement de pression fixe pour atteindre un débit souhaité.

9. Système selon l'une quelconque des revendications précédentes, comprenant en outre un capteur de pression ambiante (222).

10. Système selon la revendication 9, le système étant conçu de telle sorte que le niveau de dioxyde de carbone dans le dialysat sortant du récipient de dégazage (206) est maintenu entre 5 333 Pa et 19 998 Pa (40 mm Hg et 150 mm Hg pCO₂).
